# FASCICULE DE BREVET EUROPEEN

(11) **EP 1 256 583 B1**
(45) Date de publication et mention de la délivrance du brevet: **10.08.2005**
(21) Numéro de dépôt: 02290945.1
(22) Date de dépôt: 16.04.2002
(51) Int. Cl.: C07D 519/00, C07D 471/04, C07D 491/04, C07D 513/04, A61K 31/505

(54) **Dérivés de pyrimidin-4-ones, leur procédé de préparation et les compositions pharmaceutiques qui les contiennent**
Pyrimidin-4-one Derivate, Verfahren zu ihrer Herstellung and sie enthaltende Zusammensetzungen
Pyrimidine-4-one derivatives, process for their preparation and pharmaceutical compositions containing them

(30) Priorité: 18.04.2001 FR 0105216
(43) Date de publication de la demande: 13.11.2002
(73) Titulaire: Les Laboratoires Servier, 92415 Courbevoie Cedex (FR)
(72) Inventeur: Lavielle, Gilbert, 78170 La Celle Saint Cloud (FR); Dubuffet, Thierry, 76210 Bolbec (FR); Muller, Olivier, 95300 Pontoise (FR); Millan, Mark, 78230 Le Pecq (FR); Dekeyne, Anne, 78720 Cernay la Ville (FR); Brocco, Mauricette, 75003 Paris (FR)

(56) Documents cités:
- EP-A- 0 691 342
- EP-A- 0 887 350
- DUBUFFET T ET AL: "Novel benzopyrano[3,4-c]pyrrole derivatives as potent and selective dopamine D3 receptor antagonists" BIOORGANIC & MEDICINAL CHEMISTRY LETTERS, OXFORD, GB, vol. 9, no. 14, 19 juillet 1999 (1999-07-19), pages 2059-2064, XP004171637 ISSN: 0960-894X
- PEGLION J-L ET AL: "Tetracyclic analogues of [+]-S 14297: synthesis and determination of affinity and selectivity at cloned human dopamine D3 vs D2 receptors" BIOORGANIC & MEDICINAL CHEMISTRY LETTERS, OXFORD, GB, vol. 7, no. 7, 8 avril 1997 (1997-04-08), pages 881-886, XP004136149 ISSN: 0960-894X

## Description

La présente invention concerne de nouveaux dérivés de pyrimidin-4-ones, leur procédé de préparation et les compositions pharmaceutiques qui les contiennent.

L'invention concerne également leur utilisation en tant que ligands mixtes α₂/5-HT_{2c}.

Des dérivés de 1,2,3,3a,4,9b-hexahydrochroméno[3,4-*c*]pyrroles ont été décrits dans la demande EP 691 342 pour leur caractère antagoniste sérotoninergique, et dans la demande EP 887 350 pour leur caractère de ligand dopaminergique D₃.

Les structures corticolimbiques jouent un rôle essentiel dans les processus qui contrôlent les fonctions altérées dans les désordres psychiatriques. En particulier, il est maintenant admis que la perturbation de la transmission monoaminergique est fortement impliquée dans l'étiologie de ces différents troubles. Par exemple dans le cas de la dépression, l'activité monoaminergique est diminuée au niveau du cortex frontal, de l'hippocampe et du noyau accumbens.

Parmi les différentes classes d'auto- et hétérorécepteurs de monoamines impliqués dans les mécanismes de régulation, les récepteurs α₂-AR (autorécepteurs) et 5-HT_{2c} ont montré une importance capitale. Ces deux sous-types réceptoriels agissent dans le même sens en inhibant la transmission dopaminergique et adrénergique. D'une part un rétrocontrôle est exercé par les récepteurs α₂-AR autorécepteurs, sur les neurones noradrénergiques (J. Pharmacol. Exp. Ther., 1994, 270, 958), et d'autre part, les récepteurs α₂-AR et 5-HT_{2c} hétérorécepteurs exercent un contrôle inhibiteur sur la transmission dopaminergique et noradrénergique (Neuropharmacology, 1997, 36, 609, J. Psychopharmacol. 2000, 14 (2), 114-138).

Des composés se liant à l'un ou l'autre de ces sous-types réceptoriels ont montré leur potentiel, par le passé, dans le traitement de plusieurs pathologies.

Ainsi, le rôle bénéfique de composés antagonistes α₂-AR a été étudié dans le traitement des troubles cognitifs (J. Pharmacol., 1992, 6, 376), de la maladie de Parkinson (CNS Drugs, 1998, 10, 189), de la schizophrénie (Science 1999, 286, 105-107), de la dépression (J. Psychopharmacol. 1996, 10 Suppl. 3, 35-42), des troubles de la libido et des dysfonctionnements sexuels (J. Pharmacol., 1997, 11, 72). De la même façon, des ligands des récepteurs 5HT_{2c} ont montré leur utilité dans le traitement des dysfonctionnements sexuels (J. Pharmacol., ibid.) de la maladie de Parkinson (Drug News Perspect., 1999, 12, 477), mais aussi de l'anxiété (Br. J. Pharmacol., 1996, 117, 427), de la dépression (Pharmacol. Biochem. Behav. 1988, 29, 819-820), des désordres impulsifs (Biol. Psych. 1993, 33, 3-14), des troubles de l'appétit (British J. Pharmacol. 1998, 123, 1707-1715), des troubles du sommeil (Neuropharmacology 1994, 33 (3/4), 467-471) et de la schizophrénie (Neurosci. Lett., 1996, 181, 65).

Des composés possédant un caractère double d'antagonistes α₂-AR et 5-HT_{2c} peuvent être d'une grande utilité pour les cliniciens, afin d'obtenir, avec l'administration d'un même composé une action renforcée tout en améliorant la tolérance. Ce type de composé présente de plus un avantage considérable par rapport à l'administration de deux produits différents.

Les composés de l'invention présentent une structure originale qui leur confère ce caractère d'antagonistes doubles α₂/5-HT_{2c} et sont donc utiles dans le traitement de la dépression, des troubles du comportement impulsif, de l'anxiété, de la schizophrénie, de la maladie de Parkinson, des troubles cognitifs, des troubles de la libido, des dysfonctionnements sexuels, des troubles de l'appétit et des troubles du sommeil.

Plus spécifiquement, la présente invention concerne les composés de formule (I): 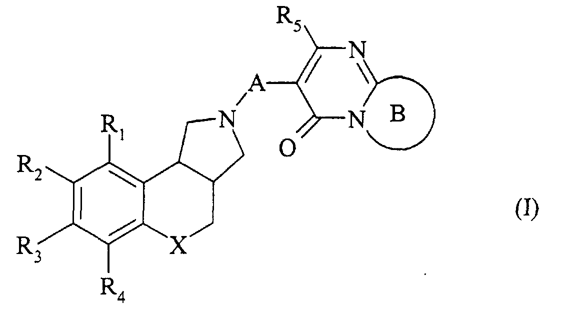 dans laquelle :
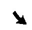 R₁, R₂, R₃ et R₄, identiques ou différents, représentent chacun un atome d'hydrogène, d'halogène ou un groupement choisi parmi alkyle (C₁-C₆) linéaire ou ramifié, alkoxy (C₁-C₆) linéaire ou ramifié, polyhalogénoalkyle (C₁-C₆) linéaire ou ramifié, hydroxy, cyano, nitro et amino,
   ou bien R₁ avec R₂, R₂ avec R₃ ou R₃ avec R₄ forment ensemble, avec les atomes de carbone qui les portent, un cycle benzénique éventuellement substitué ou hétéroaromatique éventuellement substitué,
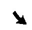 X représente un atome d'oxygène ou un groupement méthylène,
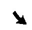 A représente une chaîne alkylène (C₁-C₆) linéaire ou ramifiée, 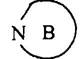
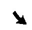 représente un hétérocycle azoté insaturé éventuellement substitué par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro, amino, phényle éventuellement substitué, thiényle éventuellement substitué, furyle éventuellement substitué et pyrrolyle éventuellement substitué,
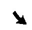 et R₅ représente un groupement alkyle (C₁-C₆) linéaire ou ramifié,
leurs énantiomères, diastéréoisomères ainsi que leurs sels d'addition à un acide ou à une base pharmaceutiquement acceptable,
étant entendu que :
- le terme alkyle (C₁-C₆) désigne une chaîne hydrocarbonée contenant de un à six atomes de carbone,
- le terme alkoxy (C₁-C₆) désigne un groupement alkyle (C₁-C₆)-oxy, contenant de un à six atomes de carbone,
- le terme alkylène (C₁-C₆) désigne une chaîne hydrocarbonée bivalente, contenant de un à six atomes de carbone,
- le terme polyhalogénoalkyle (C₁-C₆) désigne un chaîne carbonée contenant de un à six atomes de carbone et de 1 à 9 atomes d'halogène,
- le terme cycle hétéroaromatique désigne un cycle aromatique de 5 ou 6 chaînons contenant un, deux ou trois hétéroatomes choisis parmi oxygène, azote et soufre,
- le terme hétérocycle azoté insaturé désigne un cycle insaturé à une, deux ou trois insaturations, de 5 à 7 chaînons, contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote et de soufre,
- le terme éventuellement substitué affecté aux expressions cycle benzénique, cycle hétéroaromatique, phényle, thiényle, furyle ou pyrrolyle, signifie que ces groupements sont éventuellement substitués par un ou deux substituants, identiques ou différents, choisis par les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro et amino.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, méthanesulfonique, camphorique, oxalique, etc.

Parmi les bases pharmaceutiquement acceptables, on peut citer à titre non limitatif les l'hydroxyde de sodium, l'hydroxyde de potassium, la triéthylamine, etc.

Parmi les cycles hétéroaromatiques, on peut citer à titre non limitatif le thiophène, la pyridine, le furanne, le pyrrole, l'imidazole, l'oxazole, l'isoxazole, le thiazole, l'isothiazole, la pyrimidine.

Les hétérocycles azotés insaturés préférés sont la 1,2-dihydropyridine, le 2,3-dihydro-1,3-thiazole et le 2,3-dihydro-oxazole.

Par composé (3aα,9bα) ou (3aβ,9bβ), on entend composé dont la jonction de cycle correspondante est de configuration cis.

Par composé (3aα,11cα) ou (3aβ,11cβ), on entend composé dont la jonction de cycle correspondante est de configuration cis.

Par composé (3aα,9bβ) ou (3aβ,9bα), on entend composé dont la jonction correspondante de cycle est trans.

Par composé (3aα,11cβ) ou (3aβ,11cα), on entend composé dont la jonction correspondante de cycle est trans.

Dans les composés préférés de formule (I), R₁, R₂, R₃ et R₄, identiques ou différents, représentent chacun un atome d'hydrogène ou un groupement alkyle (C₁-C₆) linéaire ou ramifié, alkoxy (C₁-C₆) linéaire ou ramifié ou hydroxy.

Des composés préférés de l'invention sont ceux pour lesquels R₁ et R₂ forment ensemble avec les atomes de carbone qui les portent, un cycle benzènique et R₃, R₄ représentent chacun un atome d'hydrogène.

De façon préférentielle, X représente un atome d'oxygène.

De façon avantageuse, l'invention concerne les composés de formule (I) pour lesquels A représente une chaîne éthylène, propylène ou butylène.

Plus particulièrement, l'invention concerne les composés de formule (I) pour lesquels 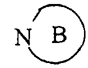 représente le groupement suivant 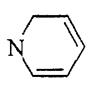 éventuellement substitué, par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro, amino et thiènyle.

D'autres composés préférés de l'invention concerne les composés de formule (I) pour lesquels 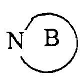 représente le groupement suivant 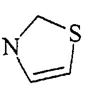 éventuellement substitué par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro et amino.

La présente invention concerne plus particulièrement les composés de formule (I) pour lesquels A représente une chaîne éthylène, X représente un atome d'oxygène, R₅ représente un groupement méthyle et 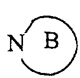 représente le groupement suivant 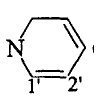 éventuellement substitué en position 2' par un atome d'halogène ou un groupement choisi parmi alkyle (C₁-C₆) linéaire ou ramifié, cyano et thiènyle.

Parmi les composés préférés de l'invention, on peut citer plus particulièrement :
- la 3-[2-((3aα,9bα)-9-méthoxy-1,3a,4,9b-tétrahydrochroméno[3,4-*c*]pyrrol-2(3*H*)-yl)-éthyl]-2-méthyl-4*H*-pyrido[1,2-*a*]pyrimidin-4-one, ainsi que ses énantiomères, diastéréoisomères et ses sels d'addition à un acide pharmaceutiquement acceptable,
- la 3-[2-((3aα,11cα)-1,3a,4,11c-tétrahydrobenzo[5,6]chroméno[3,4-*c*]pyrrol-2(3*H*)-yl)-éthyl]-2-méthyl-4*H*-pyrido[1,2-*a*]pyrimidin-4-one, ainsi que ses énantiomères et ses sels d'addition à un acide pharmaceutiquement acceptable,
- et la 3-[2-((3aβ,11cα)-1,3a,4,11c-tétrahydrobenzo[5,6]chroméno[3,4-*c*]pyrrol-2(3*H*)-yl)éthyl]-2-méthyl-4*H*-pyrido[1,2-*a*]pyrimidin-4-one, ainsi que ses énantiomères et ses sels d'addition à un acide pharmaceutiquement acceptable.

L'invention s'étend également à un procédé de préparation des composés de formule (I) caractérisé en ce que l'on soumet un composé de formule (II) : 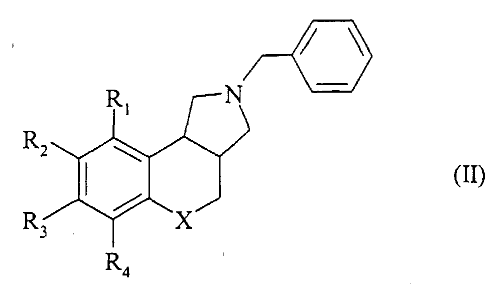 dans laquelle R₁, R₂, R₃, R₄ et X sont tels que définis dans la formule (I),
à une hydrogénation catalytique, pour conduire au composé de formule (III) : 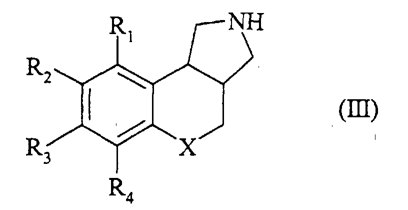 dans laquelle R₁, R₂, R₃, R₄ et X sont tels que définis précédemment,
que l'on met en réaction, soit avec un composé de formule (IV) : 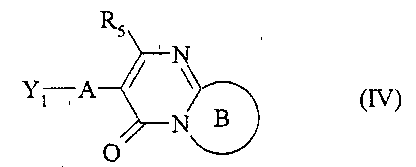 dans laquelle A, B et R₅ sont tels que définis dans la formule (I), et Y₁ représente un groupement partant tel que, par exemple, un atome d'halogène ou un groupement tosylate, triflate ou mésylate,
soit, dans des conditions d'amination réductrice, avec un composé de formule (V) : 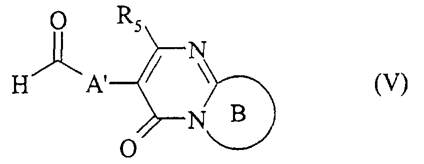 dans laquelle B et R₅ sont tels que définis dans la formule (I), et A' représente une liaison ou une chaîne alkylène (C₁-C₅) linéaire ou ramifiée,
pour conduire au composé de formule (I), que l'on purifie, le cas échéant, selon une technique classique de purification, dont on sépare, si on le souhaite, les stéréoisomères selon une technique classique de séparation, et que l'on transforme, si on le souhaite, en ses sels d'addition à un acide ou à une base pharmaceutiquement acceptable.

Les composés de formule (II) pour lesquels X représente un atome d'oxygène (composés de formule (IIa)) peuvent être obtenus à partir du composé de formule (VI) : 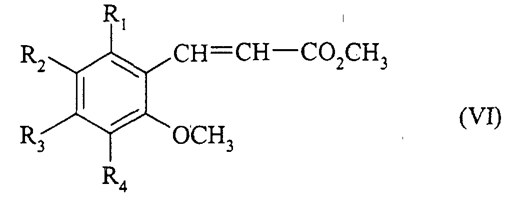 dans laquelle R₁, R₂, R₃ et R₄ sont tels que définis précédemment, sur lequel on effectue une cycloaddition avec la N-benzyl-N-(méthoxyméthyl)-triméthylsilylméthylamine, dans les conditions décrites par K. Achiwa et coll. (Chem. Pharm. Bull. 1985, 33 (7), 2762-66),
pour conduire au composé de formule (VII) : 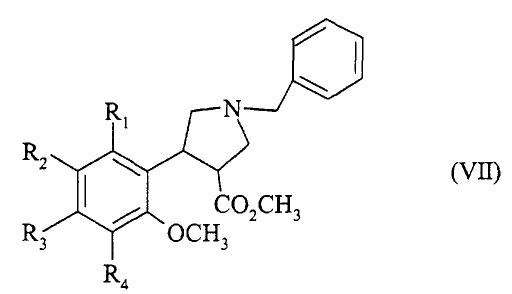 dans laquelle R₁, R₂, R₃ et R₄ sont tels que définis précédemment,
que l'on transforme ensuite, par réduction suivie de la déprotection de la fonction phénol, en composé de formule (VIII) : 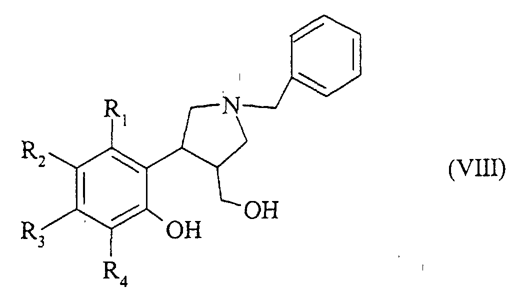 dans laquelle R₁, R₂, R₃ et R₄ sont tels que définis précédemment,
qui est ensuite transformé en composé de formule (IIa) par une réaction de Mitsunobu.

La configuration de la jonction de cycle du composé de formule (IIa) ainsi obtenu est déterminée par la configuration (Z ou E) du composé de formule (VI) utilisé.

Les composés de formule (IIa) peuvent également être obtenus à partir du composé de formule (IX) : 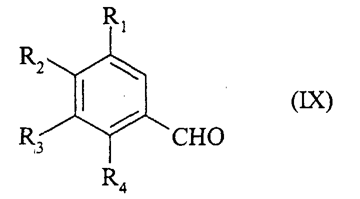 dans laquelle R₁, R₂, R₃ et R₄ sont tels que définis précédemment,
que l'on oxyde en composé de formule (X) : 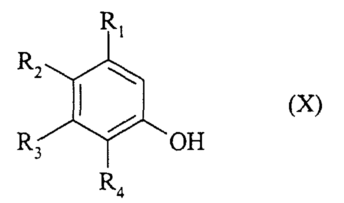 dans laquelle R₁, R₂, R₃ et R₄ sont tels que définis précédemment,
que l'on met en réaction avec le propiolate de méthyle, pour conduire à la coumarine de formule (XI) : 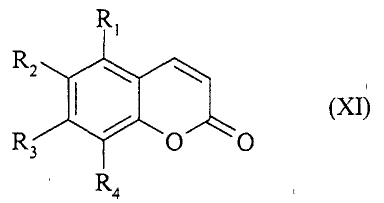 dans laquelle R₁, R₂, R₃ et R₄ sont tels que définis précédemment,
que l'on soumet :
- soit, lorsqu'on veut accéder à un composé de formule (IIa) dont la jonction de cycle est trans, à une réaction avec le méthanolate de sodium, puis à une réaction de cycloaddition avec la N-benzyl-N-(méthoxyméthyl)-triméthylsilylméthylamine, dans les conditions décrites par K. Achiwa et coll. (Chem. Pharm. Bull. 1985, 33 (7), 2762-66), suivie d'une réaction de réduction,
   pour conduire à un composé de formule (VIII) dont la configuration est trans, qui est ensuite transformé en composé de formule (IIa) trans par une réaction de Mitsunobu,
- soit, lorsqu'on veut accéder à un composé de formule (IIa) dont la jonction de cycle est cis, à une réaction de cycloaddition avec la N-benzyl-N-(méthoxyméthyl)-triméthylsilylméthylamine, dans les conditions décrites par K. Achiwa et coll. (Chem. Pharm. Bull. 1985, 33 (7), 2762-66),
   pour conduire au composé de formule (XII), dont la jonction de cycle est cis : 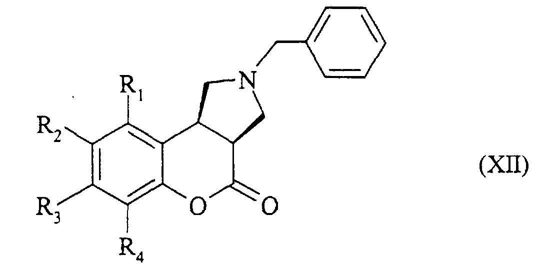
dans laquelle R₁, R₂, R₃ et R₄ sont tels que définis précédemment,
que l'on réduit ensuite en composé de formule (VIII) dont la configuration est cis, qui est ensuite transformé en composé de formule (IIa) cis par une réaction de Mitsunobu.

Les composés de formule (II) pour lesquels X représente un groupement méthylène (composés de formule (IIb)) peuvent être obtenus à partir du composé de formule (XIII) : 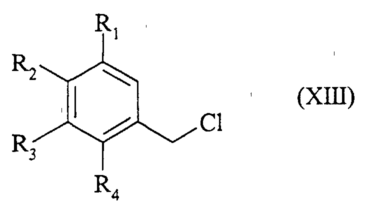 dans laquelle R₁, R₂, R₃ et R₄ sont tels que définis dans la formule (I),
que l'on fait réagir avec l'acrylate d'éthyle pour conduire au composé de formule (XIV) : 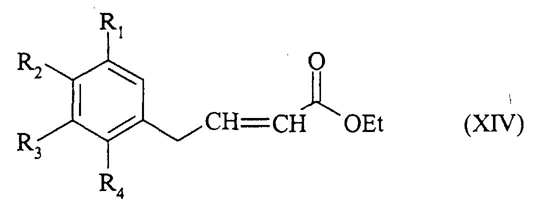 dans laquelle R₁, R₂, R₃ et R₄ sont tels que définis précédemment,
que l'on transforme, par réduction suivie d'une saponification, en composé de formule (XV) : 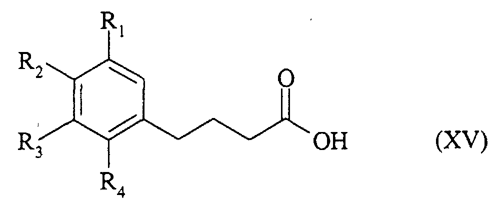 dans laquelle R₁, R₂, R₃ et R₄ sont tels que définis précédemment,
que l'on cyclise en composé de formule (XVI) : 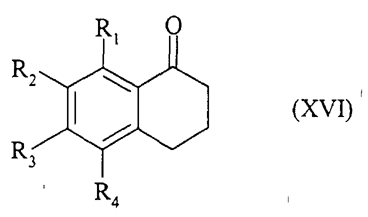 dans laquelle R₁, R₂, R₃ et R₄ sont tels que définis précédemment,
que l'on transforme ensuite en composé de formule (XVII) : 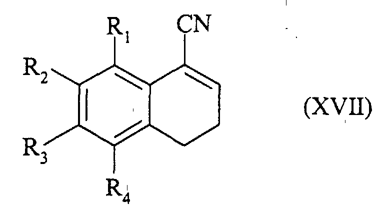 dans laquelle R₁, R₂, R₃ et R₄ sont tels que définis précédemment,
puis, par addition de cyanure de lithium, en composé de formule (XVIII) : 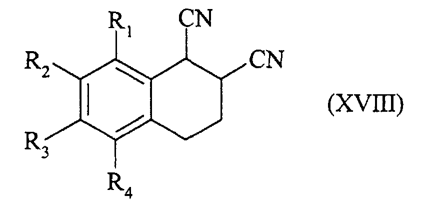 dans laquelle R₁, R₂, R₃ et R₄ sont tels que définis précédemment,
dont on transforme les groupements cyano en groupements carboxy, avant de les condenser pour conduire au composé de formule (XIX) : 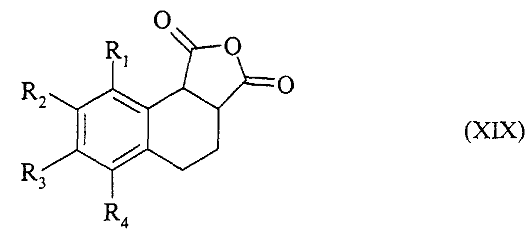 dans laquelle R₁, R₂, R₃ et R₄ sont tels que définis précédemment,
que l'on fait ensuite réagir avec la benzylamine, pour conduire au composé de formule (XX) : 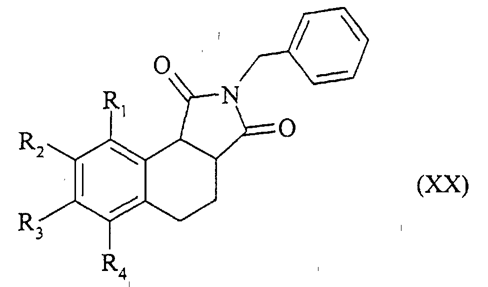 dans laquelle R₁, R₂, R₃ et R₄ sont tels que définis précédemment,
que l'on transforme par réduction en composé de formule (IIb).

L'invention s'étend aussi aux compositions pharmaceutiques renfermant comme principe actif un composé de formule (I) avec un ou plusieurs excipients inertes, non toxiques et pharmaceutiquement acceptables. Parmi les compositions pharmaceutiques selon l'invention, on pourra citer plus particulièrement celles qui conviennent pour l'administration orale, parentérale (intraveineuse, intramusculaire ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient et les traitements éventuellement associés. Cette posologie varie de 0,5 mg à 2 g par 24 heures en une ou plusieurs prises.

Les exemples suivants illustrent l'invention.

Les produits de départ utilisés sont des produits connus ou préparés selon des modes préparatoires connus.

Les structures des composés décrits dans les exemples ont été déterminées selon les techniques spectrométriques usuelles (infrarouge, RMN, spectrométrie de masse).

### EXEMPLE 1 : 3-[2-((3aα,9bα)-9-Méthoxy-1,3a,4,9b-tétrahydrochroméno[3,4-c] pyrrol-2(3H)-yl)-éthyl]-2-méthyl-4H-pyrido[1,2-a]pyrimidin-4-one, dichlorhydrate

### Stade A : (3α,4α)-1-Benzyl-4-(2,6-diméthoxyphényl)-3-pyrrolidine-carboxylate de méthyle

A une solution, refroidie à 5°C, contenant 120 mmoles de l'ester méthylique de l'acide cis-(2,6-diméthoxy)cinnamique, et 0,1 ml d'acide trifluoroacétique dans 150 ml d'acétate d'éthyle, sont ajoutées lentement 100 mmoles de N-benzyl-N-(méthoxyméthyl) triméthylsilylméthylamine. Le milieu réactionnel est porté de 30°C à 55°C en 75 minutes. 0,75 g de carbonate de potassium sont alors ajoutés et l'ensemble est maintenu sous agitation 15 minutes. Après filtration et évaporation des solvants, le résidu est repris par 100 ml d'acétate d'éthyle et la solution portée à 50°C. 110 mmoles d'acide oxalique en solution dans 100 ml d'acétone sont alors ajoutées sous vive agitation. Celle-ci est maintenue 15 heures. Après filtration puis lavage à l'éther, l'oxalate obtenu est traité par deux équivalents de potasse 1N pour conduire au produit attendu.

### Stade B : (3α,4α)-1-Benzyl-3-hydroxyméthyl-4-(2,6-diméthoxyphényl)-pyrrolidine

A 120 mmoles d'hydrure de lithium-aluminium en suspension dans 800 ml de tétrahydrofurane à +5 °C sont ajoutées 100 mmoles du composé obtenu au stade précédent en solution dans 400 ml de tétrahydrofurane. Après une heure d'agitation à cette température, le milieu est traité par une addition lente de 11 ml d'eau, 15 ml de soude 2N et 26 ml d'eau. Le milieu est agité 10 heures puis filtré. Le solvant est évaporé pour conduire au produit attendu.

### Stade C : (3α,4α)-1-Benzyl-3-hydroxyméthyl-4-(2,6-dihydroxyphényl)-pyrrolidine

A 8,9 mmoles du composé obtenu au stade précédent dans 170 ml de dichlorométhane, sont ajoutés 44,5 ml d'une solution 1M de tribromure de bore dans le dichlorométhane. Le milieu réactionnel est porté 8 heures à reflux puis traité par de la soude concentrée pendant une heure. Le milieu est alors neutralisé à l'aide d'acide chlorhydrique. Après extraction au dichlorométhane, le produit attendu est obtenu après purification du résidu par chromatographie sur silice (éluant : dichlorométhane/méthanol 95/5).

### Stade D : (3aα,9bα)-2-Benzyl-9-hydroxy-1,2,3,3a,4,9b-hexahydrochroméno[3,4-c] pyrrole, chlorhydrate

A 80 mmoles du composé obtenu au stade précédent dans 1 litre de tétrahydrofurane sont additionnées successivement 120 mmoles de diéthylazodicarboxylate et 120 mmoles de triphénylphosphine. Le milieu est agité à température ambiante pendant 15 heures. Le solvant est évaporé et le produit brut est repris dans 1 litre d'éther isopropylique et porté au reflux pendant une heure. Le précipité obtenu est éliminé et le filtrat est concentré avant d'être chromatographié (éluant : cyclohexane/acétate d'éthyle 70/30). Le produit obtenu est salifié par l'acide chlorhydrique.

### Stade E : (3aα,9bα)-2-Benzyl-9-méthoxy-1,2,3,3a,4,9b-hexahydrochroméno[3,4-c] pyrrole, chlorhydrate

11,6 mmoles du composé obtenu au stade précédent dans 50 ml de diméthylformamide sont ajoutées à une solution contenant 14 mmoles d'hydrure de sodium dans 50 ml de diméthylformamide. Après 30 minutes d'agitation, on ajoute 11,6 mmoles d'iodure de méthyle. Après 1 heure à température ambiante puis hydrolyse, les solvants sont évaporés. Le résidu est alors repris à l'eau. Après extraction à l'éther, séchage et évaporation, le produit attendu est obtenu après purification du résidu par chromatographie sur colonne de silice en utilisant comme éluant un mélange cyclohexane/acétate d'éthyle (75/25).

| *Microanalyse élémentaire :* | | | | |
|---|---|---|---|---|
| | *C%* | *H%* | *N%* | *Cl%* |
| *calculé* | *68,77* | *6,68* | *4,22* | *10,68* |
| *trouvé* | *68,66* | *4,48* | *4,45* | *10,97* |

### Stade F : (3aα,9bα)-9-Méthoxy-1,2,3,3a,4,9b-hexahydrochroméno[3,4-c]pyrrole

50 mmoles du composé obtenu au stade précédent est mis en solution dans un mélange de 200 ml de méthanol et 50 ml d'eau. 50 mmoles d'une solution 5 N d'acide chlorhydrique dans l'éthanol sont ajoutées. Après addition de 2 grammes de dihydroxyde de palladium, le milieu réactionnel est mis sous atmosphère d'hydrogène. Après absorption du volume théorique d'hydrogène, le catalyseur est filtré et le filtrat est évaporé. Le résidu obtenu est traité par de la soude 1 N et extrait par le dichlorométhane pour conduire au produit attendu.

### Stade G : 3-[2-((3aα,9bα)-9-Méthoxy-1,3a,4,9b-tétrahydrochroméno[3,4-c] pyrrol-2(3H)-yl)-éthyl]-2-méthyl-4H-pyrido[1,2-a]pyrimidin-4-one, dichlorhydrate

A 7 mmoles du composé obtenu au stade précédent dans 100 ml d'acétonitrile sont additionnées 7 mmoles de carbonate de potassium. Le milieu est porté à 60°C. 7 mmoles de 3-(2-chloroéthyl)-2-méthylpyrido[1,2-a]pyrimidin-4-one (préparée selon Liebigs Ann. Chem. 1973, 103-110) sont alors additionnées en une fois. Le milieu est porté à 80°C pendant 18 heures. Après refroidissement et addition d'eau, le milieu est extrait au dichlorométhane puis lavé et séché. Après filtration et évaporation des solvants, le résidu obtenu est purifié par chromatographie sur silice (éluant : dichlorométhane/méthanol 95/5). Le produit obtenu est salifié par l'acide chlorhydrique.

| *Microanalyse élémentaire :* | | | | |
|---|---|---|---|---|
| | *C%* | *H%* | *N%* | *Cl%* |
| *calculé* | *59,49* | *5,86* | *9,05* | *15,27* |
| *trouvé* | *59,57* | *5,89* | *9,09* | *15,41* |

### EXEMPLE 2 : 3-[2-((3aα,9bα)-6,8-Diméthoxy-7-méthyl-1,3a,4,9b-tétrahydrochroméno[3,4-c]pyrrol-2(3H)-yl)-éthyl]-2-méthyl-4H-pyrido[1,2-a] pyrimidin-4-one, sesquifumarate

### Stade A : 2,4-Diméthoxy-3-méthyl-phénol

A 416 mmoles de 2,4-diméthoxy-3-méthyl-benzaldéhyde en solution dans 200ml de dichlorométhane sont ajoutées 624 mmoles d'acide méta-chloroperbenzoique. Le milieu réactionnel est porté au reflux pendant 5 heures avant d'être filtré. Le filtrat est lavé par 3 fois 200ml d'une solution saturée de NaHCO₃ puis séché sur sulfate de magnésium. Après évaporation du solvant, le produit brut est repris par 400 ml de méthanol. 624 mmoles d'une solution de KOH à 10% sont ajoutées. Le milieu est agité une heure puis est amené à pH=4 par une solution 1N d'acide chlorhydrique. Après une heure d'agitation, le volume est réduit à 1/3. Le produit brut est repris par 400ml d'eau et extrait par du dichlorométhane. Les phases organiques sont collectées et séchées. Après évaporation des solvants, le résidu obtenu est purifié par chromatographie sur colonne de silice (éluant : cyclohexane/acétate d'éthyle 90/10) pour conduire au produit attendu.

### Stade B : 6,8-Diméthoxy-7-méthyl-coumarine

A 270 mmoles du composé obtenu au stade précédent en solution dans 500 ml d'acide méthanesulfonique anhydre sont ajoutées 270 mmoles de propiolate de méthyle. Le milieu réactionnel est porté à 90 °C pendant 30 minutes puis refroidi à 0°C. Après une addition lente d'eau, le milieu est extrait au dichlorométhane. Les phases organiques sont collectées, lavées par une solution 1 N de soude puis séchées. Après évaporation des solvants, le résidu obtenu est trituré dans l'éther éthylique puis filtré pour conduire au produit attendu.

### Stade C : (3α,4α)-1-Benzyl-3-hydroxyméthyl-4-(3,5-diméthoxy-2-hydroxy-4-tolyl)-pyrrolidine

Le produit attendu est obtenu selon le procédé décrit dans les stades A et B de l'exemple 1 à partir du composé obtenu au stade précédent.

### Stade D : (3aα,9bα)-2-Benzyl-6,8-diméthoxy-7-méthyl-1,2,3,3a,4,9b-hexahydrochroméno[3,4-c]pyrrole, chlorhydrate

Le produit attendu est obtenu selon le procédé décrit dans le stade D de l'exemple 1 à partir du composé obtenu au stade précédent.

| *Microanalyse élémentaire :* | | | | |
|---|---|---|---|---|
| | *C%* | *H%* | *N%* | *Cl%* |
| *calculé* | *67,10* | *6,97* | *3,73* | *9,43* |
| *trouvé* | *66,90* | *7,00* | *3,92* | *9,57* |

### Stade E : (3aα,9bα)-6,8-Diméthoxy-7-méthyl-1,2,3,3a,4,9b-hexahydrochroméno [3,4-c]pyrrole

Le produit attendu est obtenu selon le procédé décrit dans le stade F de l'exemple 1 à partir du composé obtenu au stade précédent.

### Stade F : 3-[2-((3aα,9bα)-6,8-Diméthoxy-7-méthyl-1,3a,4,9b-tétrahydrochroméno [3,4-c]pyrrol-2(3H)-yl)-éthyl]-2-méthyl-4H-pyrido[1,2-a]pyrimidin-4-one, sesquifumarate

Le produit attendu est obtenu selon le procédé décrit dans le stade G de l'exemple 1 à partir du composé obtenu au stade précédent. Le produit obtenu est salifié par l'acide fumarique.

| *Microanalyse élémentaire :* | | | |
|---|---|---|---|
| | *C%* | *H%* | *N%* |
| *calculé* | *61,07* | *5,80* | *6,89* |
| *trouvé* | *61,73* | *6,03* | *7,19* |

### EXEMPLE 3 : 3-[2-(1,3a,4,11c-Tétrahydrobenzo[5,6]chroméno[3,4-c] pyrrol-2(3H)-yl)-éthyl]-2-méthyl-4H-pyrido[1,2-a]pyrimidin-4-one, fumarate

### Stade A : 1,2,3,3a,4,11c-Hexahydrobenzo[5,6]chroméno[3,4-c]pyrrole

Le produit attendu est obtenu selon le procédé décrit dans les stades C à F de l'exemple 2 à partir de benzo[*f*]chromèn-3-one.

### Stade B : 3-[2-(1,3a,4,11c-Tétrahydrobenzo[5,6]chroméno[3,4-c]pyrrol-2(3H)-yl)-éthyl]-2-méthyl-4H-pyrido[1,2-a]pyrimidin-4-one, fumarate

Le produit attendu est obtenu selon le procédé décrit dans le stade G de l'exemple 1 à partir du composé obtenu au stade précédent.

| *Microanalyse élémentaire :* | | | |
|---|---|---|---|
| | *C%* | *H%* | *N%* |
| *calculé* | *68,30* | *5,54* | *7,96* |
| *trouvé* | *67,87* | *5,57* | *7,79* |

### EXEMPLE 4 : 6-[2-((3aα,11cα)-1,3a,4,11c-Tétrahydrobenzo[5,6]chroméno[3,4-c] pyrrol-2(3H)-yl)-éthyl]-7-méthyl-5H-[1,3]thiazolo[3,2-a]pyrimidin-5-one, fumarate

Le produit attendu est obtenu selon le procédé décrit dans le stade G de l'exemple 1, en utilisant comme produits de départ le composé décrit au stade A de l'exemple 3 et la 6-(2-chloro-éthyl)-7-méthyl-thiazolo[3,2-*a*]pyrimidin-5-one. Le produit obtenu est salifié par l'acide fumarique.

| *Microanalyse élémentaire :* | | | | |
|---|---|---|---|---|
| | *C%* | *H%* | *N%* | *S%* |
| *calculé* | *63,03* | *5,10* | *7,87* | *6,01* |
| *trouvé* | *62,91* | *5,08* | *7,77* | *5,83* |

### EXEMPLE 5 : 3-[3-((3aα,11cα)-1,3a,4,11c-Tétrahydrobenzo[5,6]chroméno[3,4-c] pyrrol-2(3H)-yl)propyl]-2-méthyl-4H-pyrido[1,2-a]pyrimidin-4-one, hémifumarate

### Stade A : 3-(2-Méthyl)-4-oxo-pyrido[1,2-a]pyrimidin-3-yl)-propionaldéhyde

A 60 mmoles de 3-(2-chloro-éthyl)-2-méthyl-pyrido[1,2-α]pyrimidin-4-one (préparée selon Liebigs Ann. Chem. 1973, 103-110) dans 140 ml de diméthylsulfoxyde sont ajoutées 120 mmoles de cyanure de potassium. Le milieu réactionnel est porté à 100°C pendant 3 heures. Après évaporation du diméthylsulfoxyde, le produit brut est repris par du dichlorométhane et de l'eau, extrait au dichlorométhane. Les phases organiques sont lavées et séchées. Le résidu est purifié par chromatographie sur silice (éluant : dichlorométhane/méthanol 95/5) puis le produit obtenu est mis en solution dans 100 ml de dichlorométhane. Le milieu est refroidi à -70°C, 120 mmoles d'hydrure de diisobutylaluminium sont introduites. Après 2 heures d'agitation à cette température, le milieu est traité par 50 ml de méthanol et 100 ml d'eau, puis la phase organique est lavée et séchée pour conduire au produit attendu.

### Stade B : 3-[3-((3aα,11cα)-1,3a,4,11c-Tétrahydrobenzo[5,6]chroméno[3,4-c]pyrrol-2(3H)-yl)propyl]-2-méthyl-4H-pyrido[1,2-α]pyrimidin-4-one, hémifumarate

A 10 mmoles du composé décrit au stade A de l'exemple 3 en solution dans le 1,2-dichloroéthane sont ajoutées 10 mmoles du composé obtenu au stade précédent, puis 14 mmoles de triacétoxyborohydrure de sodium. Après 4 heures d'agitation, de l'eau est ajoutée, puis le milieu réactionnel est décanté et extrait au dichlorométhane. Les phases organiques rassemblées sont séchées, puis filtrées et évaporées. Le résidu ainsi obtenu est purifié par chromatographie sur silice (éluant : dichlorométhane/méthanol 95/5), pour conduire, après salification par l'acide fumarique, au produit attendu.

| *Microanalyse élémentaire :* | | | |
|---|---|---|---|
| | *C%* | *H%* | *N%* |
| *calculé* | *72,03* | *6,04* | *8,69* |
| *trouvé* | *71,68* | *5,95* | *8,59* |

### EXEMPLE 6 : 3-{2-[(3aα,11cα)-1,3,3a,4,5,11c-Hexahydro-2H-naphto[1,2-e]isoindol-2-yl]éthyl}-2-méthyl-4H-pyrido[1,2-a]pyrimidin-4-one, fumarate

### Stade A : 2-Chlorométhyl-naphthalène

A 1,26 mole de naphthalèn-2-yl-méthanol dans 1,5 litre de toluène sont ajoutées lentement 3,8 moles de chlorure de thionyle. Le milieu est ensuite porté au reflux pendant deux heures. Après refroidissement, le solvant est évaporé. Le produit brut est repris deux fois au toluène et évaporé. L'huile obtenue est reprise au dicholorométhane, lavée et séchée. Après filtration et évaporation, l'huile obtenue est distillée pour conduire au produit attendu.
*Point d'ébullition : 118°C sous 0,09 mm Hg*

### Stade B : 4-(2-Naphtyl)-2-buténoate d'éthyle

A 863 mmoles du composé obtenu au stade précédent dans 206 ml de tri-butylamine sont ajoutées 1,07 mole d'acrylate d'éthyle et 10 mmoles d'acétate de palladium. Le milieu réactionnel est porté au reflux pendant cinq heures. Après refroidissement, le milieu est hydrolysé et extrait une fois par du dichlorométhane. La phase organique est lavée par une solution d'acide chlorhydrique 1N puis par de l'eau avant d'être séchée. Le produit est purifié par chromatographie sur silice (éluant : cyclohexane/acétate d'éthyle 95/5) pour conduire au produit attendu.

### Stade C : 4-(2-Naphtyl)-2-butanoate d'éthyle

A 490 mmoles du composé obtenu au stade précédent dans 3 1 d'éthanol, sont ajoutés 7 grammes de palladium sur charbon à 10%. Le milieu est mis sous atmosphère d'hydrogène. Après absorption de la quantité théorique d'hydrogène, le milieu est filtré et le solvant est évaporé pour conduire au produit attendu.

### Stade D : Acide 4-(2-naphtyl)-2-butanoïque

A 484 mmoles du composé obtenu au stade précédent dans 1,5 litre d'éthanol, sont additionnés 490 ml d'une solution titrée de soude 2 N. Le milieu est porté et maintenu au reflux pendant une heure. L'éthanol est ensuite évaporé. Le pH est amené à une valeur de 5-6 par de l'acide chlorhydrique 1 N. Le précipité formé est filtré et lavé à l'eau pour conduire au produit attendu.

### Stade E : 2,3-Dihydro-4(1H)-phénanthrénone

A 550 g d'acide polyphosphorique sont ajoutées 240 mmoles du composé obtenu au stade précédent. Le milieu réactionnel est chauffé à 80°C pendant 3 heures puis versé sur de la glace. Après une heure d'agitation, le précipité formé est filtré, lavé à l'eau et séché pour conduire au produit attendu.

### Stade F : 1,2-Dihydro-4-phénanthrène-carbonitrile

A 207 mmoles du composé obtenu au stade précédent dans 500 ml de tétrahydrofurane sont additionnées successivement 243 mmoles de cyanure de triméthylsilane puis 16 mmoles d'une solution 0,5 M de cyanure de lithium dans la diméthylformamide. Le milieu réactionnel est agité pendant 2 heures puis versé sur de la glace et extrait 4 fois à l'éther. Les phases organiques sont collectées et lavées par de l'eau avant d'être séchées. Après filtration, les solvants sont évaporés. Le résidu est mis en solution dans 325 ml de pyridine. 42 ml de POCl₃ sont additionnés. Le milieu est porté à 100 °C pendant 3 heures puis refroidi avant d'être versé sur 400 ml d'acide chlorhydrique glacé. Après addition de dichlorométhane, le milieu est décanté, puis extrait au dichlorométhane. Les phases organiques rassemblées sont lavées puis séchées. Après filtration, les solvants sont évaporés. Le résidu obtenu est purifié par chromatographie sur silice (éluant: cyclohexane/dichlorométhane : 70/30) pour conduire au produit attendu.

### Stade G : 1,2,3,4-Tetrahydro-phénanthrène-3,4-dicarbonitrile

A 87 mmoles d'acide acétique sont additionnées 105 mmoles d'une solution 0,5 M de cyanure de lithium, puis, à 5 °C, 82 mmoles du composé obtenu au stade précédent en solution dans 500 ml de diméthylformamide. Le milieu réactionnel est porté à 100°C pendant deux heures avant d'être refroidi puis séché et versé sur de la glace. Le précipité obtenu est filtré et lavé pour conduire au produit attendu.

### Stade H : Acide 1,2,3,4-tétrahydro-phénanthrène-3,4-dicarboxylique

A 68 mmoles du composé obtenu au stade précédent sont additionnées successivement 42 ml d'acide acétique puis 84 ml d'acide chlorhydrique concentré. Le milieu réactionnel est porté au reflux pendant 5 jours avant d'être refroidi et versé sur un litre d'eau. Le produit est extrait à l'acétate d'éthyle. Les phases organiques sont collectées et séchées. Après filtration et évaporation du solvant, le résidu obtenu est purifié par chromatographie (éluant : dichlorométhane/méthanol/acide acétique : 95/5/0,5) pour conduire au produit attendu.

### Stade I : (3aα,11cα)-3a,4,5,11c-Tétrahydrophénantro[3,4-c]furan-1,3-dione

A 37 mmoles du composé obtenu au stade précédent sont additionnées 1,67 moles d'anhydride acétique. Le milieu réactionnel est porté au reflux pendant 2 h puis évaporé à sec. Le résidu obtenu est trituré dans l'éther pour conduire au produit attendu sous la forme de cristaux.

### Stade J : (3aα,11cα)-2-Benzyl-3a,4,5,11c-tétrahydro-1H-naphto[1,2-e]isoindole-1,3(2H)-dione

A 18 mmoles du composé obtenu au stade précédent dans 400 ml de toluène sont additionnées 18 mmoles de benzylamine. Le milieu réactionnel est porté au reflux pendant 30 heures. 70 ml d'une solution 0,1 N d'acide chlorhydrique sont ajoutés. Le milieu est décanté. La phase organique est séchée, filtrée puis évaporée. Le résidu obtenu est purifié par chromatographie sur silice (éluant: dichlorométhane/cyclohexane : 60/40) pour conduire au produit attendu.

### Stade K : (3aα, 11cα)- 2-Benzyl-2,3,3a,4,5,11c-hexahydro-1H-naphto[1,2-e] isoindole, chlorhydrate

Sur 93 mmoles d'hydrure d'aluminium-lithium, sont versés 180 ml d'éther anhydre. Après refroidissement à 10°C, 15,5 mmoles du composé obtenu au stade précédent en solution dans 250 ml de dichlorométhane sont ajoutées. Le milieu réactionnel est agité à température ambiante pendant 2 heures puis est refroidi à -10°C. 90 ml d'eau sont additionnés lentement. Le gel obtenu est filtré et lavé abondamment au dichlorométhane. Les phases organiques et le filtrat sont rassemblés et décantés. La phase organique est séchée, filtrée puis évaporée. Le résidu obtenu est purifié par chromatographie sur silice (éluant : cyclohexane/acétate d'éthyle 80/20). Le produit obtenu est salifié par l'acide chlorhydrique.

| *Microanalyse élémentaire :* | | | | |
|---|---|---|---|---|
| | *C%* | *H%* | *N%* | *Cl%* |
| *calculé* | *78,95* | *6,91* | *4,00* | *10,13* |
| *trouvé* | *78,60* | *6,99* | *4,28* | *10,04* |

### Stade L : 3-{2-[(3aα,11cα)-1,3,3a,4,5,11c-Hexahydro-2H-naphto[1,2-e]isoindol-2-yl]éthyl}-2-méthyl-4H-pyrido[1,2-a]pyrimidin-4-one, fumarate

Le produit attendu est obtenu selon le procédé décrit dans les stades F et G de l'exemple 1 à partir du composé obtenu au stade précédent. Le produit obtenu est salifié par l'acide fumarique.

| *Microanalyse élémentaire :* | | | |
|---|---|---|---|
| | *C%* | *H%* | *N%* |
| *calculé* | *70,49* | *5,88* | *7,85* |
| *trouvé* | *70,84* | *5,94* | *7,99* |

### EXEMPLE 7 : 3-[2-((3aα,9bβ)-6,8-Diméthoxy-7-méthyl-1,3a,4,9b-tétrahydrochroméno[3,4-c]pyrrol-2(3H)-yl)-éthyl]-2-méthyl-4H-pyrido[1,2-a] pyrimidin-4-one, sesquifumarate

### Stade A : Ester méthylique de l'acide trans-(3,5-diméthoxy-6-hydroxy-6-méthyl)-cinnamique

A 70 mmoles du composé obtenu au stade B de l'exemple 2 dans 100 ml de méthanol sont ajoutées lentement 205 mmoles d'une solution à 30 % de méthylate de sodium dans le méthanol. Le milieu réactionnel est porté au reflux pendant 4 heures avant d'être hydrolysé par une solution d'acide chlorhydrique 1 N. Le précipité est filtré, lavé à l'eau et séché pour conduire au produit attendu.

### Stade B : (3aα,9bβ)-2-Benzyl-6,8-diméthoxy-7-méthyl-1,2,3,3a,4,9b-hexahydrochroméno[3,4-c]pyrrole, clilorhydrate

Le produit attendu est obtenu selon le procédé décrit dans les stades C et D de l'exemple 2 à partir du composé obtenu au stade précédent.

| *Microanalyse élémentaire :* | | | | |
|---|---|---|---|---|
| | *C%* | *H%* | *N%* | *Cl%* |
| *calculé* | *67,10* | *6,97* | *3,73* | *9,43* |
| *trouvé* | *67,15* | *7,13* | *3,76* | *9,73* |

### Stade C : 3-[2-((3aα,9bβ)-6,8-Diméthoxy-7-méthyl-1,3a,4,9b-tétrahydrochroméno[3,4-c]pyrrol-2(3H)-yl)-éthyl]-2-méthyl-4H-pyrido[1,2-a] pyrimidin-4-one, sesquifumarate

Le produit attendu est obtenu selon le procédé décrit dans les stades F et G de l'exemple 1 à partir du composé obtenu au stade précédent. Le produit obtenu est salifié par l'acide fumarique.

### EXAMPLE 8 : 3-[2-(1,3a,4,11c-Tétrahydrobenzo[5,6]chroméno[3,4-c]pyrrol-2(3H)-yl)éthyl]-7-chloro-2-méthyl-4H-pyrido[1,2-a]pyrimidin-4-one, fumarate

### Stade A : 7-Chloro-3-(2-chloroéthyl)-2-méthyl-4H-pyrido[1,2-a]pyrimidin-4-one

Le produit est préparé selon une protocole décrit dans la littérature (Liebigs Ann. Chem. 1973, 103-110) en utilisant la 2-amino-5-chloropyridine comme réactif de départ.

### Stade B : 3-[2-(1,3a,4,11c-Tétrahydrobenzo[5,6]chroméno[3,4-c]pyrrol-2(3H)-yl) éthyl]-7-chloro-2-méthyl-4H-pyrido[1,2-a]pyrimidin-4-one, fumarate

Le produit attendu est obtenu selon le procédé décrit dans le stade G de l'exemple 1 en utilisant le composé décrit au stade précédent et le composé obtenu au stade A de l'exemple 3 (le produit est salifié par l'acide fumarique).

| *Microanalyse élémentaire :* | | | | |
|---|---|---|---|---|
| | *C%* | *H%* | *N%* | *Cl%* |
| *calculé* | *63,21* | *4,97* | *7,18* | *6,08* |
| *trouvé* | *62,98* | *5,08* | *7,03* | *6,19* |

### EXEMPLE 9 : 3-[2-((3aα,9bα)-6,8-Diméthoxy-7-méthyl-1,3a,4,9b-tétrahydrochroméno[3,4-c]pyrrol-2(3H)-yl)éthyl]-7-chloro-2-méthyl-4H-pyrido[1,2-a]pyrimidin-4-one, fumarate

Le produit attendu est obtenu selon le procédé décrit dans l'exemple 2 en utilisant comme produit de départ la 7-chloro-3-(2-chloroéthyl)-2-méthyl-4*H*-pyrido[1,2-α]pyrimidin-4-one préparée au stade A de l'exemple 8, à la place de la 3-(2-chloroéthyl)-2-méthyl-4*H*-pyrido[1,2-*a*]pyrimidin-4-one. Le produit obtenu est salifié par l'acide fumarique.

| *Microanalyse élémentaire :* | | | | |
|---|---|---|---|---|
| | *C%* | *H%* | *N%* | *Cl%* |
| *calculé* | *59,44* | *5,5* | *7,17* | *6,05* |
| *trouvé* | *58,94* | *5,43* | *6,97* | *6,34* |

### EXEMPLE 10: 6-[2-((3aα,9bα)-6-Méthoxy-1,3a,4,9b-tétrahydrochroméno[3,4-c] pyrrol-2(3H)-yl)éthyl]-7-méthyl-5H-[1,3]thiazolo[3,2-a]pyrimidin-5-one, fumarate

### Stade A : (3aα,9bα)-6-Méthoxy-1,2,3,3a,4,9b-hexahydrochroméno[3,4-c]pyrrole

Le produit est préparé comme aux stades A-F de l'exemple 1 à partir de l'acide cis-(2,3 diméthoxy)cinnamique.

### Stade B : 6-[2-((3aα,9bα)-6-Méthoxy-1,3a,4,9b-tétrahydrochroméno[3,4-c]pyrrol-2(3H)-yl)éthyl]-7-méthyl-5H-[1,3]thiazolo[3,2-a]pyrimidin-5-one, fumarate

Le produit attendu est obtenu à partir du composé préparé au stade précédent suivant le mode opératoire utilisé dans le stade G de l'exemple 1 en utilisant la 6-(2-chloroéthyl)-7-méthyl-5*H*-[1,3]thiazolo[3,2-*a*]pyrimidin-5-one. Le produit est salifié par l'acide fumarique.

| *Microanalyse élémentaire :* | | | | |
|---|---|---|---|---|
| | *C%* | *H%* | *N%* | *S%* |
| *calculé* | *58,47* | *5,30* | *8,18* | *6,24* |
| *trouvé* | *58,78* | *5,22* | *8,27* | *6,20* |

### EXEMPLE 11 : 3-[2-((3aα,9bα)-6-Méthoxy-1,3a,4,9b-tétrahydrochroméno[3,4-c] pyrrol-2(3H)-yl)éthyl]-2-méthyl-4H-pyrido[1,2-a]pyrimidin-4-one, fumarate

Le produit attendu est obtenu selon le procédé décrit dans l'exemple 1, stade G en utilisant comme produits départ le composé décrit au stade A de l'exemple 10 et le 3-(2-chloroéthyl)-2-méthyl-4*H*-pyrido[1,2-*a*]pyrimidin-4-one. Le produit obtenu est salifié par l'acide fumarique.

| *Microanalyse élémentaire :* | | | |
|---|---|---|---|
| | *C%* | *H%* | *N%* |
| *calculé* | *63,90* | *5,76* | *8,28* |
| *trouvé* | *63,03* | *5,77* | *7,91* |

### EXEMPLE 12 : 3-[2-((3aα,9bα)-6-Méthoxy-1,3a,4,9b-tétrahydrochroméno[3,4-c] pyrrol-2(3H)-yl)éthyl]-7-bromo-2-méthyl-4H-pyrido[1,2-a] pyrimidin-4-one, fumarate

### Stade A : 3-(2-Chloroéthyl)-7-bromo-2-méthyl-4H-pyrido-[1,2-a]pyrimidin-4-one

Le produit est préparé selon le procédé décrit dans le stade A de l'exemple 8 en utilisant la 2-amino-5-bromopyridine comme réactif de départ.

### Stade B : 3-[2-((3aα,9bα)-6-Méthoxy-1,3a,4,9b-tétrahydrochroméno[3,4-c]pyrrol-2(3H)-yl)éthyl]-7-bromo-2-méthyl-4H-pyrido[1,2-a]pyrimidin-4-one. fumarate

Le produit attendu est obtenu selon le procédé décrit dans l'exemple 11 à partir du composé de l'exemple 10 et de la pyrimidinone préparé au stade précédent. Le produit est salifié par l'acide fumarique.

| *Microanalyse élémentaire :* | | | | |
|---|---|---|---|---|
| | *C%* | *H%* | *N%* | *Br%* |
| *calculé* | *55,30* | *4,81* | *7,17* | *13,63* |
| *trouvé* | *55,02* | *4,75* | *7,12* | *13,81* |

### EXEMPLE 13 : 3-[2-((3aα,11cα)-1,3a,4,11c-Tétrahydrobenzo[5,6]chroméno[3,4-c] pyrrol-2(3H)-yl)éthyl]-2,7-diméthyl-4H-pyrido[1,2-a]pyrimidin-4-one, fumarate

### Stade A : 3-(2-Chloroéthyl)-2,7-diméthyl-4H-pyrido[1,2-a]pyrimidin-4-one

Le produit est préparé selon le procédé décrit dans le stade A de l'exemple 8 en utilisant la 2-amino-5-méthylpyridine comme réactif de départ.

### Stade B : 3-[2-((3aα,11cα)-1,3a,4,11c-Tétrahydrobenzo[5,6]chroméno[3,4-c]pyrrol-2(3H)-yl)éthyl]-2,7-diméthyl-4H-pyrido[1,2-a]pyrimidin-4-one, fumarate

Le produit attendu est obtenu selon le procédé décrit dans le stade G de l'exemple 1 à partir du composé préparé au stade précédent et du composé du stade A de l'exemple 3. Le produit est salifié par l'acide fumarique.

| *Microanalyse élémentaire :* | | | |
|---|---|---|---|
| | *C%* | *H%* | *N%* |
| *calculé* | *68,75* | *5,77* | *7,76* |
| *trouvé* | *67,96* | *5,60* | *7,67* |

### EXEMPLE 14 : 3-[2-((3aα,9bα)-6,8-Diméthoxy-7-méthyl-1,3a,4,9b-tétrahydrochroméno[3,4-c]pyrrol-2(3H)-yl)éthyl]-2,7-diméthyl-4H-pyrido [1,2-a]pyrimidin-4-one, fumarate

Le produit attendu est obtenu selon le procédé décrit dans l'exemple 1, stade G en utilisant comme produits départ le composé obtenu au stade A de l'exemple 13 et le produit du stade E de l'exemple 2. Le produit est salifié par l'acide fumarique.

| *Microanalyse élémentaire* | | | |
|---|---|---|---|
| | *C%* | *H%* | *N%* |
| *calculé* | *63,70* | *6, 24* | *7,43* |
| *trouvé* | *63,57* | *6,09* | *7,36* |

### EXEMPLE 15 : 3-[2-((3aα,11cα)-1,3a,4,11c-Tétrahydrobenzo[5,6]chroméno[3,4-c] pyrrol-2(3H)-yl)éthyl]-7-bromo-2-méthyl-4H-pyrido[1,2-a] pyrimidin-4-one, fumarate

Le produit attendu est obtenu selon le procédé décrit dans l'exemple 1, stade G en utilisant comme produits départ le composé obtenu stade A de l'exemple 3 avec le produit obtenu au stade A de l'exemple 12. Le produit est salifié par l'acide fumarique.

| *Microanalyse élémentaire :* | | | | |
|---|---|---|---|---|
| | *C%* | *H%* | *N%* | *Br%* |
| *calculé* | *59,41* | *4,65* | *6,93* | *13,18* |
| *trouvé* | *59,04* | *4,69* | *6,81* | *12,93* |

### EXEMPLE 16 : 3-[2-((3aα,9bα)-6-Hydroxy-8-méthoxy-7-méthyl-1,3a,4,9b-tétrahydrochroméno[3,4-c]pyrrol-2(3H)-yl)éthyl]-2-méthyl-4H-pyrido[1,2-a]pyrimidin-4-one, fumarate

### Stade A : (3aα,9bα)-2-Benzyl-8-méthoxy-7-méthyl-1,2,3,3a,4,9b-hexahydrochroméno [3,4-c]pyrrol-6-ol

Ajouter à 0 °C, 64 ml de solution molaire dans le dichlorométhane de tribromure de bore à une solution de 20 g de composé décrit au stade D de l'exemple 2. Après trois heures d'agitation à 20 °C, hydrolyser par 200 ml de solution saturée d'hydrogénocarbonate de sodium et décanter la phase organique. Le résidu obtenu est purifié par chromatographie sur gel de silice en utilisant comme éluant un mélange dichlorométhane/méthanol : 98/2.

### Stade B : (3aα,9bα)-8-Méthoxy-7-méthyl-1,2,3,3a,4,9b-hexahydrochromeno[3,4-c] pyrrol-6-ol

Le produit préparé au stade précédent est débenzylé en appliquant le mode opératoire décrit au stade F de l'exemple 1.

### Stade C : 3-[2-((3aα,9bα)-6-Hydroxy-8-méthoxy-7-méthyl-1,3a,4,9b-tétrahydrochroméno[3,4-c]pyrrol-2(3H)-yl)éthyl]-2-méthyl-4H-pyrido[1,2-a] pyrimidin-4-one, fumarate

Le produit attendu est obtenu selon le procédé décrit au stade G de l'exemple 1 en utilisant comme réactif de départ le composé décrit au stade précédent et la 3-(2-chloroéthyl)-2-méthyl-4*H*-pyrido[1,2-*a*]pyrimidin-4-one. Le produit est salifié par l'acide fumarique.

| *Microanalyse élémentaire :* | | | |
|---|---|---|---|
| | *C%* | *H%* | *N%* |
| *calculé* | *62,56* | *5,81* | *7,82* |
| *trouvé* | *61,61* | *5,97* | *7,42* |

### EXEMPLE 17 : 3-[4-(3aα,11cα)-1,3a,4,11c-Tétrahydrobenzo[5,6]chroméno[3,4-c] pyrrol-2(3H)-yl)butyl]-2-méthyl-4H-pyrido[1,2-a]pyrimidin-4-one, fumarate

### Stade A : 3-(3-Butènyl)-2-méthyl-4H-pyrido[1,2-a]pyrimidin-4-one

Porter 2 heures à 160 °C un mélange de 5,7 g de 2-acétyl-5-hexènoate de méthyle, 3,15 g de 2-aminopyridine et 0,65 g d'acide polyphosphorique. Puis additionner à froid 30 ml d'eau et extraire le tout à l'aide de dichlorométhane. Le résidu obtenu après évaporation du solvant est purifié par chromatographie sur gel de silice en utilisant comme éluant un mélange dichlorométhane/méthanol : 98/2.

### Stade B : 3-Butyl-2-méthyl-4H-pyrido[1,2-a]pyrimidin-4-one

A une solution de 5,4 g du produit obtenu à l'étape précédente dans 100 ml de tétrahydrofurane ajouter 55,2 ml d'une solution molaire de borane dans le tétrahydrofurane. Après une heure refroidir à 0 °C et additionner 10 ml d'eau puis 25,4 g de borate de sodium. Laisser agiter une heure puis concentrer le tout, reprendre le résidu dans 100 ml d'eau et extraire à l'aide de dichlorométhane. Après évaporation du solvant le résidu obtenu est purifié par chromatographie sur gel de silice en utilisant comme éluant un mélange dichlorométhane/méthanol : 98/2.

### Stade C : 4-(2-Méthyl-4-oxo-4H-pyrido[1,2-a]pyrimidin-3-yl)butanal

Porter 3 h à reflux un mélange de 4,5 g du produit obtenu à l'étape précédente avec 8,1 g de 1-hydroxy-1-oxo-benzo[*d*][1,2]iodoxol-3-one dans 200 ml de tétrahydrofurane. Filtrer à froid et concentrer le filtrat.

### Stade D : 3-[4-(3aα,11cα)-1,3a,4,11c-Tétrahydrobenzo[5,6]chroméno[3,4-c]pyrrol-2(3H)-yl)butyl]-2-méthyl-4H-pyrido[1,2-a]pyrimidin-4-one

La suite de la synthèse est réalisée en appliquant le mode opératoire décrit au stade B de l'exemple 5 à partir de l'aldéhyde obtenu au stade précédent.
Le produit est salifié par l'acide fumarique

| *Microanalyse élémentaire :* | | | |
|---|---|---|---|
| | *C%* | *H%* | *N%* |
| *calculé* | *68,06* | *5,89* | *7,26* |
| *trouvé* | *67,25* | *5,85* | *6,82* |

### EXEMPLE 18 : 3-[2-((3aα,11cα)-1,3a,4,11c-Tétrahydrobenzo[5,6]chroméno[3,4-c] pyrrol-2(3H)-yl)éthyl]-2-méthyl-4H-pyrido[1,2-a]pyrimidin-4-one, fumarate

### Stade A : (3aα,11cα)-1,2,3,3a,4,11c-Hexahydrobenzo[5,6]chroméno[3,4-c]pyrrole

Dédoublement du racémate 1,2,3,3a,4,11c-hexahydrobenzo[5,6]chroméno[3,4-*c*]pyrrole : Dissoudre 53,6 g de produit obtenu au stade A de l'exemple 3 dans 450 ml d'éthanol et porter le tout au reflux. Ajouter une solution de 85,2 g d'acide (+)2,3-dibenzoyl-D-tartrique dans 450 ml d'éthanol. Filtrer le précipité 12 heures plus tard. Recristalliser ce précipité trois fois dans un mélange éthanol/eau : 85/15. Le composé est obtenu sous forme de base après traitement à la soude aqueuse.

### Stade B : 3-[2-((3aα,11cα)-1,3a,4,11c-Tétrahydrobenzo[5,6]chroméno[3,4-c]pyrrol-2(3H)-yl)éthyl]-2-méthyl-4H-pyrido[1,2-a]pyrimidin-4-one, fumarate

Le produit attendu est obtenu selon le procédé décrit au stade G de l'exemple 1 en utilisant comme produits de départ l'énantiomère préparé au stade précédent et le 3-(2-chloroéthyl)-2-méthyl-4*H*-pyrido[1,2-*a*]pyrimidin-4-one.
Le produit est salifié par l'acide fumarique

| *Microanalyse élémentaire :* | | | |
|---|---|---|---|
| | *C%* | *H%* | *N%* |
| *calculé* | *68,30* | *5,54* | *7,96* |
| *trouvé* | *68,24* | *5,41* | *8,12* |

### EXEMPLE 19 : 3-[2-((3aβ,11cβ)-1,3a,4,11c-Tétrahydrobenzo[5,6]chroméno[3,4-c] pyrrol-2(3H)-yl)éthyl]-2-méthyl-4H-pyrido[1,2-a]pyrimidin-4-one, fumarate

### Stade A : (3aβ,11cβ)-1,2,3,3a,4,11c-Hexahydrobenzo[5,6]chroméno[3,4-c]pyrrole

Dédoublement du racémate : 1,2,3,3a,4,11c-hexahydrobenzo[5,6]chroméno[3,4-*c*]pyrrole Dissoudre 10,3 g de produit obtenu au stade A de l'exemple 3 dans 200 ml d'éthanol et porter le tout au reflux. Ajouter une solution de 16,4 g d'acide (-)2,3-dibenzoyl-L-tartrique dans 200 ml d'éthanol. Filtrer le précipité 12 heures plus tard. Recristalliser ce précipité trois fois dans un mélange éthanol/eau : 85/15. Le composé est obtenu sous forme de base après traitement à la soude aqueuse.

### Stade B : 3-[2-((3aβ,11cβ)-1,3a,4,11c-Tétrahydrobenzo[5,6]chroméno[3,4-c]pyrrol-2(3H)-yl)éthyl]-2-méthyl-4H-pyrido[1,2-a]pyrimidin-4-one, fumarate

Le produit attendu est obtenu selon le procédé décrit au stade G de l'exemple 1 en utilisant comme produits de départ l'énantiomère préparé au stade précédent et le 3-(2-chloroéthyl)-2-méthyl-4*H*-pyrido[1,2-*a*]pyrimidin-4-one.
Le produit est salifié par l'acide fumarique

| *Microanalyse élémentaire :* | | | |
|---|---|---|---|
| | *C%* | *H%* | *N%* |
| *calculé* | *68,30* | *5,54* | *7,96* |
| *trouvé* | *68,85* | *5,45* | *7,81* |

### EXEMPLE 20 : 3-[2-((3aα,9bα)-6,8-Diméthoxy-7-propyl-1,3a,4,9b-tétrahydrochroméno[3,4-c]pyrrol-2(3H)-yl)éthyl]-2-méthyl-4H-pyrido[1,2-a] pyrimidin-4-one, fumarate

### Stade A : 2-Allyloxy-4-méthoxybenzaldéhyde

A une solution de 100 g de 2-hydroxy-4-méthoxy benzaldéhyde dans 2 1 d'acétone ajouter 79,5 ml de bromure d'allyle puis progressivement 272 g de carbonate de potassium. Porter 4 heures à reflux puis filtrer, concentrer sous vide le filtrat, reprendre le résidu par 1 1 de dichlorométhane et laver cette solution à l'aide d'une solution d'hydroxyde de sodium normale puis par une solution de chlorure de sodium saturée. Sécher sur sulfate de magnésium et concentrer sous vide.

### Stade B : 3-Allyl-2-hydroxy-4-méthoxybenzaldéhyde

Chauffer à 200 °C pendant 15 heures une solution de 125 g du produit obtenu au stade A dans 300 ml de *N,N*-diméthylaniline. Evaporer ensuite le solvant sous vide, reprendre le résidu dans 1 1 d'éther isopropylique, extraire cette solution par 10 fois avec 100 ml de solution d'hydroxyde de sodium normale, puis acidifier l'ensemble de ces extraits à l'aide d'acide chlorhydrique 3 N. Extraire alors la solution aqueuse acide par du dichlorométhane, sécher et concentrer la phase organique.

### Stade C : 3-Allyl-2,4-diméthoxybenzaldehyde

Porter 5 heures à reflux un mélange de 108 g du produit obtenu au stade précédent, 74,4 g de sulfate de diméthyle, 233 g de carbonate de potassium dans 2 1 d'acétone. Filtrer à froid et concentrer la solution obtenue. Le résidu est repris dans l'éther isopropylique, lavé par une solution saturée d'hydrogénocarbonate de sodium puis à l'eau. Sécher sur sulfate de magnésium et évaporer le solvant sous vide. Le résidu est purifié par chromatographie sur gel de silice en utilisant comme éluant un mélange cyclohexane/acétate d'éthyle : 96/4.

### Stade D : 2,4-Diméthoxy-3-propylbenzaldehyde

Hydrogéner à température ambiante et à sous pression atmosphérique 64 g du produit obtenu au stade C à l'aide de 35 g de catalyseur de Wilkinson (chlorure de *tris*-triphénylphosphine rhodium) dans 500 ml de benzène. Le produit est purifié par chromatographie sur gel de silice en utilisant comme éluant un mélange cyclohexane/acétate d'éthyle : 92/8.

### Stade E : 3-[2-((3aα,9bα)-6,8-Diméthoxy-7-propyl-1,3a,4,9b-tétrahydro chroméno[3,4-c]pyrrol-2(3H)-yl)éthyl]-2-méthyl-4H-pyrido[1,2-a] pyrimidin-4-one, fumarate

La suite de la synthèse est réalisée en appliquant les modes opératoires décrits dans l'exemple 2 (stades A à F) en utilisant l'aldéhyde obtenu au stade précédent comme réactif de départ.
Le produit est salifié par l'acide fumarique.

| *Microanalyse élémentaire* | | | |
|---|---|---|---|
| | *C%* | *H%* | *N%* |
| *calculé* | *64,24* | *6,43* | *7,25* |
| *trouvé* | *63,33* | *6,30* | *7,05* |

### EXEMPLE 21 : 3-[2-((3aα,11cα)-1,3a,4,11c-Tétrahydrobenzo[5,6]chroméno[3,4-c] pyrrol-2(3H)-yl)éthyl]-7-chloro-2-méthyl-4H-pyrido[1,2-a]pyrimidin-4-one, fumarate

### Stade A : (7-Chloro-2-méthyl-4-oxo-4H-pyrido[1,2-a]pyrimidin-3-yl)acétaldéhyde

Le produit est préparé en suivant le mode opératoire décrit au stade C de l'exemple 18 en utilisant le 7-chloro-3-(2-hydroxyéthyl)-2-méthyl-4*H*-pyrido[1,2-*a*]pyrimidin-4-one comme substrat de départ obtenu suivant un procédé décrit dans la littérature (Liebigs Ann. Chem. 1973, 103-110).

### Stade B : 3-[2-((3aα,11cα)-1,3a,4,11c-Tétrahydrobenzo[5,6]chroméno]3,4-c]pyrrol-2(3H)-yl)éthyl]-7-chloro-2-méthyl-4H-pyrido[1,2-a]pyrimidin-4-one, fumarate

Le produit attendu est obtenu selon le procédé décrit au stade B de l'exemple 5 en utilisant le composé préparé au stade A de l'exemple 19 et l'aldéhyde préparé au stade précédent. Le produit est salifié par l'acide fumarique.

| *Microanalyse élémentaire :* | | | | |
|---|---|---|---|---|
| | *C%* | *H%* | *N%* | *Cl%* |
| *calculé* | *64,11* | *5,02* | *7,48* | *6,31* |
| *trouvé* | *63,82* | *5,02* | *7,35* | *6,63* |

### EXEMPLE 22 : 3-[2-((3aβ,11cβ)-1,3a,4,11c-Tétrahydrobenzo[5,6]chroméno[3,4-c] pyrrol-2(3H)-yl)éthyl]-7-chloro-2-méthyl-4H-pyrido[1,2-a]pyrimidin-4-one, fumarate

Le produit attendu est obtenu selon le procédé décrit au stade B de l'exemple 5 en utilisant le composé préparé au stade A de l'exemple 20 et l'aldéhyde préparé au stade A de l'exemple 22. Le produit est salifié par l'acide fumarique.

| *Microanalyse élémentaire* | | | | |
|---|---|---|---|---|
| | *C%* | *H%* | *N%* | *Cl%* |
| *calculé* | *64,11* | *5,02* | *7,48* | *6,31* |
| *trouvé* | *64,30* | *4,92* | *7,53* | *6,56* |

### EXEMPLE 23 : 3-[2-((3aα,11cα)-1,3a,4,11c-Tétrahydrobenzo[5,6]chroméno[3,4-c] pyrrol-2(3H)-yl)éthyl]-2-méthyl-4-oxo-4H-pyrido[1,2-a]pyrimidine-7-carbonitrile, fumarate

A une solution de 1,83 mmol du composé obtenu dans l'exemple 15 dans 12 ml de diméthylformamide est ajouté, sous atmosphère inerte, 1,1 mmol de cyanure de zinc et 0,073 mmol de tétrakis triphénylphospine de palladium. Chauffer le milieu réactionnel 12 heures à 90 °C. Evaporer le milieu réactionnel, reprendre au chlorure de méthylène, filtrer sur célite puis évaporer. Le résidu obtenu est purifié par chromatographie sur silice (éluant : dichlorométhane/méthanol : 95/5). Le produit est salifié par l'acide fumarique.

| *Microanalyse élémentaire :* | | | |
|---|---|---|---|
| | *C%* | *H%* | *N%* |
| *calculé* | *68,52* | *5,18* | *10,58* |
| *trouvé* | *68,63* | *5,05* | *10,58* |

### EXEMPLE 24 : 3-[2-((3aα,11cα)-1,3a,4,11c-Tétrahydrobenzo[5,6]chroméno[3,4-c] pyrrol-2(3H)-yl)éthyl]-2-méthyl-7-(2-thiènyl)-4H-pyrido[1,2-a] pyrimidin-4-one, fumarate

### Stade A : 3-(2-Hydroxyéthyl)-2-méthyl-7-(2-thiènyl)-4H-pyrido[1,2-a]pyrimidin-4-one

Mélanger dans un ballon sous courant d'argon 5 g de 7-bromo-3-(2-hydroxyéthyl)-2-méthyl-4*H*-pyrido[1,2-*a*]pyrimidin-4-one, 6,2 ml de tributyl-thiophèn-2-yl-stannane, 1 g de tetrakis triphénylphosphine de palladium et 250 ml de *N*-méthylpyrrolidone. Porter cette solution à 130°C pendant 2 heures puis évaporer le solvant sous vide, reprendre le résidu dans 200 ml de dichlorométhane, laver la solution avec une solution de fluorure de potassium à 10%, filtrer, décanter la phase organique et la laver à l'eau. Après séchage sur sulfate de magnésium et évaporation, le résidu obtenu est purifié par chromatographie sur gel de silice en utilisant comme éluant un mélange dichlorométhane/ méthanol : 95/5.

### Stade B : 3-(2-Chloroéthyl)-2-méthyl-7-(2-thiènyl)-4H-pyrido[1,2-a]pyrimidin-4-one

Le produit du stade précédent est transformé en dérivé chloré en appliquant un mode opératoire décrit dans la littérature (Liebigs Ann.Chem. 1973, 103-110).

### Stade C : 3-[2-((3aα,11cα)-1,3a,4,11c-Tétrahydrobenzo[5,6]chroméno[3,4-c]pyrrol-2(3H)-yl)éthyl]-2-méthyl-7-(2-thiènyl)-4H-pyrido[1,2-a]pyrimidin-4-one, fumarate

La suite de la synthèse est réalisée en appliquant le mode opératoire décrit dans l'exemple 1 (stade G) en utilisant le produit obtenu au stade précédent à la place du 3-(2-chloroéthyl)-2-méthylpyrido[1,2-*a*]pyrimidin-4-one. Le produit est salifié par l'acide fumarique.

| *Microanalyse élémentaire :* | | | | |
|---|---|---|---|---|
| | *C%* | *H%* | *N%* | *S%* |
| *calculé* | *66,98* | *5,12* | *6,89* | *5,26* |
| *trouvé* | *66,02* | *5,07* | *6,85* | *4,94* |

### EXEMPLE 25 : 3-[2-((3aβ,11cα)-1,3a,4,11c-Tétrahydrobenzo[5,6]chroméno[3,4-c] pyrrol-2(3H)-yl)éthyl]-7-chloro-2-méthyl-4H-pyrido[1,2-a]pyrimidin-4-one, hémifumarate

### Stade A : (3aβ,11cα)-1,2,3,3a,4,11c-Hexahydrobenzo[5,6]chroméno[3,4-c]pyrrole

Ce composé est obtenu suivant le même protocole expérimental que celui décrit dans la demande de brevet EP 691 342 (exemple 33).

### Stade B : 3-[2-((3aβ,11cα)-1,3a,4,11c-Tétrahydrobenzo[5,6]chroméno[3,4-c]pyrrol-2(3H)-yl)éthyl]-7-chloro-2-méthyl-4H-pyrido[1,2-a]pyrimidin-4-one, hémifumarate

Le produit attendu est obtenu selon le procédé décrit dans le stade G de l'exemple 1 en utilisant le composé décrit au stade précédent et le composé obtenu au stade A de l'exemple 8 (le produit est salifié par l'acide fumarique).

| *Microanalyse élémentaire :* | | | | |
|---|---|---|---|---|
| | *C%* | *H%* | *N%* | *Cl%* |
| *calculé* | *66,16* | *5,16* | *8,15* | *6,88* |
| *trouvé* | *66,33* | *5,10* | *8,31* | *7,40* |

### EXEMPLE 26 : 6-[2-((3aβ,11cα)-1,3a,4,11c-Tétrahydrobenzo[5,6]chroméno[3,4-c] pyrrol-2(3H)-yl)éthyl]-7-méthyl-5H-[1,3]thiazolo[3,2-a]pyrimidin-5-one, hémifumarate

Le produit attendu est obtenu selon le procédé décrit au stade G de l'exemple 1 en utilisant le composé obtenu au stade A de l'exemple 26 avec la 6-(2-chloroéthyl)-7-méthyl-5*H*-[1,3]thiazolo[3,2-*a*]pyrimidin-5-one (le produit est salifié par l'acide fumarique).

| *Microanalyse élémentaire :* | | | | |
|---|---|---|---|---|
| | *C%* | *H%* | *N%* | *Cl%* |
| *calculé* | *65,09* | *5,26* | *8,63* | *6,58* |
| *trouvé* | *64,98* | *5,28* | *8,61* | *6,67* |

### EXEMPLE 27 : 3-[2-((3aβ,11cα)-1,3a,4,11c-Tétrahydrobenzo[5,6]chroméno[3,4-c] pyrrol-2(3H)-yl)éthyl]-2-méthyl-4H-pyrido[1,2-a]pyrimidin-4-one, hémifumarate

Le produit attendu est obtenu selon le procédé décrit dans le stade G de l'exemple 1 en utilisant le composé décrit au stade A de l'exemple 26 avec la 3-(2-chloroéthyl)-2-méthylpyrido[1,2-*a*]pyrimidin-4-one (le produit est salifié par l'acide fumarique).

| *Microanalyse élémentaire :* | | | |
|---|---|---|---|
| | *C%* | *H%* | *N%* |
| *calculé* | *70,90* | *5,74* | *8,73* |
| *trouvé* | *70,99* | *5,70* | *8,77* |

### ETUDE PHARMACOLOGIQUE

### EXEMPLE A : Test des érections péniennes chez le rat

Le test permet d'évaluer la capacité d'agents pharmacologiques à inhiber les érection péniennes provoquées par l'administration d'un agoniste sélectif 5-HT_{2c}, le RO 60-0175.

Des rats mâles de souche Wistar pesant 120-140 g le jour de l'expérience, sont placé individuellement dans des boîtes d'observation en plexiglass, juste après avoir reçu le composé à tester ou le véhicule. Trente minutes plus tard, les animaux reçoivent le RO 60-0175 (1,25 mg/kg, sous-cutané) et le nombre d'érections effectuées lors des 30 minutes suivantes est comptabilisé.

*Résultats* *:* Il apparaît que les composés de l'invention sont capables d'inhiber les érections péniennes provoquées par l'administration de l'agoniste sélectif 5-HT_{2c}. Ils possèdent donc un caractère antagoniste au niveau des récepteurs 5-HT_{2c}. A titre d'exemple, le composé de l'exemple 3 possède une dose inhibitrice 50 (ID₅₀) de 2,6 mg/kg, s.c.

### EXEMPLE B : Test d'agressivité induite par isolement chez la souris

Les animaux utilisés sont des souris mâles CD-1. Dès leur arrivée, les souris sont isolées en cages individuelles avec nourriture et boisson à volonté. Après une période d'un mois d'isolement, des couples de souris stables en agressivité sont sélectionnés en observant, lorsqu'elles sont mises en présence, la latence, le nombre et la durée des attaques.

Le test a lieu une fois par semaine. Le jour du test chaque souris du couple (résidente et intruse) reçoit une injection intra-péritonéale du véhicule (animaux contrôles) ou de produit à tester (animaux traités) à un volume de 10 ml/kg. Après 30 minutes, la souris intruse est introduite dans la cage de la souris résidente. La latence de la première attaque, le nombre et la durée des attaques sont alors mesurées pendant une période de trois minutes.
Un produit est considéré comme spécifiquement antiagressif lorsqu'il diminue le nombre et la durée des attaques à des doses non sédatives.

*Résultats* *:* Il apparaît que les composés de l'invention diminuent de façon significative le nombre et la durée des attaques. A titre d'exemple, le composé de l'exemple 3 possède une dose inhibitrice 50 (ID₅₀) de 7 mg/kg, i.p..

### EXEMPLE C : Test d'enfouissement des billes chez la souris

Le test permet d'évaluer la capacité d'agents pharmacologiques à inhiber le comportement spontané d'enfouissement de billes chez la souris, cette inhibition étant prédictive d'actions antidépressive et/ou anti-impulsive.

Des souris mâles de souche NMRI pesant 20 à 25 g le jour de l'expérience, sont placées individuellement dans des boîtes en macrolon contenant 5 cm de sciure et recouvertes par une plaque en plexiglass perforée. Vingt-quatre billes en verre "oeil de chat" sont réparties régulièrement sur la sciure à la périphérie de la boîte. Au terme de 30 minutes d'exploration libre, les animaux sont retirés de la boîte et le nombre de billes enfouies est comptabilisé.

*Résultats* *:* Il apparaît que les composés de l'invention inhibent le comportement spontané d'enfouissement de billes chez la souris. A titre d'exemple, le composé de l'exemple 3 possède une dose inhibitrice 50 (ID₅₀) de 4,1 mg/kg, s.c.

### EXEMPLE D : Détermination de l'affinité pour les récepteurs α₂ adrénergiques chez le rat

L'affinité a été déterminée par des expériences de compétition avec le [³H]-RX 821,002. Les membranes sont préparées à partir de cortex cérébral de rat et incubées en triple avec 0.4 nM de [³H]-RX 821,002 et le composé à tester dans un volume final de 1,0 ml, pendant 60 minutes à 22°C. Le tampon d'incubation contient 50 nM de TRIS-HCl (pH 7,5), 1 mM de EDTA et 100 µM de GppNHp. La fixation non spécifique est déterminée avec 10 µM de phentolamine.

*Analyse de données :* A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est détermine par comptage du liquide de scintillation. Les isothermes de binding sont analysées par régression non-linéaire.

*Résultats* *:* Les composés de l'invention interagissent de façon spécifique avec les récepteurs α₂-adrénergiques avec, par exemple pour le composé de l'exemple 3 un pKᵢ de 7,5.

### EXEMPLE E : Composition Pharmaceutique

### Formule de préparation pour 1000 comprimés dosés à 10 mg

| | |
|---|---|
| Composé de l'exemple 3 | 10 g |
| Hydroxypropylcellulose | 2 g |
| Amidon de blé | 10 g |
| Lactose | 10 g |
| Stéarate de magnésium | 3 g |
| Talc | 3 g |

## Revendications

1. Composés de formule (I): 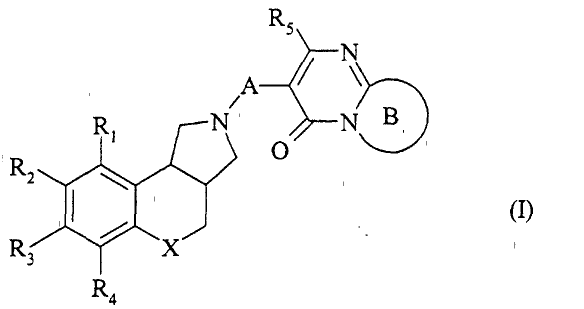 dans laquelle :
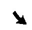 R₁, R₂, R₃ et R₄, identiques ou différents, représentent chacun un atome d'hydrogène, d'halogène ou un groupement choisi parmi alkyle (C₁-C₆) linéaire ou ramifié, alkoxy (C₁-C₆) linéaire ou ramifié, polyhalogénoalkyle (C₁-C₆) linéaire ou ramifié, hydroxy, cyano, nitro et amino,
ou bien R₁ avec R₂, R₂ avec R₃ ou R₃ avec R₄ forment ensemble, avec les atomes de carbone qui les portent, un cycle benzénique éventuellement substitué ou hétéroaromatique éventuellement substitué,
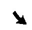 X représente un atome d'oxygène ou un groupement méthylène,
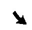 A représente une chaîne alkylène (C₁-C₆) linéaire ou ramifiée, 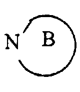
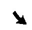 représente un hétérocycle azoté insaturé éventuellement substitué par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro, amino, phényle éventuellement substitué, thiényle éventuellement substitué, furyle éventuellement substitué et pyrrolyle éventuellement substitué, et R₅ représente un groupement alkyle (C₁-C₆) linéaire ou ramifié,
leurs énantiomères, diastéréoisomères ainsi que leurs sels d'addition à un acide ou à une base pharmaceutiquement acceptable,
étant entendu que :
- le terme alkyle (C₁-C₆) désigne une chaîne hydrocarbonée contenant de un à six atomes de carbone,
- le terme alkoxy (C₁-C₆) désigne un groupement alkyle (C₁-C₆)-oxy, contenant de un à six atomes de carbone,
- le terme alkylène (C₁-C₆) désigne une chaîne hydrocarbonée bivalente, contenant de un à six atomes de carbone,
- le terme polyhalogénoalkyle (C₁-C₆) désigne un chaîne carbonée contenant de un à six atomes de carbone et de 1 à 9 atomes d'halogène,
- le terme cycle hétéroaromatique désigne un cycle aromatique de 5 ou 6 chaînons contenant un, deux ou trois hétéroatomes choisis parmi oxygène, azote et soufre,
- le terme hétérocycle azoté insaturé désigne un cycle insaturé à une, deux ou trois insaturations, de 5 à 7 chaînons, contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote et de soufre,
- le terme éventuellement substitué affecté aux expressions cycle benzénique, cycle hétéroaromatique, phényle, thiényle, furyle ou pyrrolyle, signifie que ces groupements sont éventuellement substitués par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro et amino.

2. Composés de formule (I) selon la revendication 1, pour lesquels R₁, R₂, R₃ et R₄, identiques ou différents, représentent chacun un atome d'hydrogène ou un groupement alkyle (C₁-C₆) linéaire ou ramifié, alkoxy (C₁-C₆) linéaire ou ramifié ou hydroxy, ainsi que leurs énantiomères, diastéréoisomères et leurs sels d'addition à un acide ou à une base pharmaceutiquement acceptable.

3. Composés de formule (I) selon la revendication 1, pour lesquels R₁ et R₂ forment ensemble avec les atomes de carbone qui les portent, un cycle benzènique, et R₃, R₄ représentent chacun un atome d'hydrogène, ainsi que leurs énantiomères, diastéréoisomères et leurs sels d'addition à un acide ou à une base pharmaceutiquement acceptable.

4. Composés de formule (I) selon l'une quelconque des revendications 1 à 3, pour lesquels X représente un atome d'oxygène, ainsi que leurs énantiomères, diastéréoisomères et leurs sels d'addition à un acide ou à une base pharmaceutiquement acceptable.

5. Composés de formule (I) selon l'une quelconque des revendications 1 à 4, pour lesquels A représente une chaîne éthylène, propylène ou butylène, ainsi que leurs énantiomères, diastéréoisomères et leurs sels d'addition à un acide ou à une base pharmaceutiquement acceptable.

6. Composés de formule (I) selon l'une quelconque des revendications 1 à 5, pour lesquels 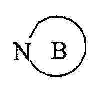 représente le groupement suivant 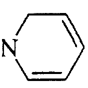 éventuellement substitué par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro, amino et thiènyle, ainsi que leurs énantiomères, diastéréoisomères et leurs sels d'addition à un acide ou à une base pharmaceutiquement acceptable.

7. Composés de formule (I) selon l'une quelconque des revendications 1 à 5, pour 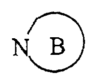 représente le groupement suivant 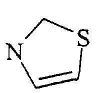 éventuellement substitué par un ou deux substituants, identiques ou différents, choisis parmi les atomes d'halogène et les groupements alkyle (C₁-C₆) linéaire ou ramifié, hydroxy, alkoxy (C₁-C₆) linéaire ou ramifié, polyhalogénoalkyle (C₁-C₆) linéaire ou ramifié, cyano, nitro et amino, ainsi que leurs énantiomères, diastéréoisomères et leurs sels d'addition à un acide ou à une base pharmaceutiquement acceptable.

8. Composés de formule (I) selon l'une quelconque des revendications 1 à 6, pour lesquels A représente une chaîne éthylène, X représente un atome d'oxygène, R₅ représente un groupement méthyle et 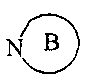 représente le groupement suivant 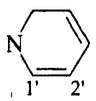 éventuellement substitué en position 2' par un atome d'halogène ou un groupement choisi parmi alkyle (C₁-C₆) linéaire ou ramifié, cyano et thiènyle ainsi que leurs énantiomères, diastéréoisomères et leurs sels d'addition à un acide ou à une base pharmaceutiquement acceptable.

9. Composé de formule (I) selon l'une quelconque des revendications 1 à 2, 4 à 6 et 8 qui est la 3-[2-((3aα,9bα)-9-méthoxy-1,3a,4,9b-tétrahydrochroméno[3,4-*c*]pyrrol-2(3*H*)-yl)-éthyl]-2-méthyl-4*H*-pyrido[1,2-*a*]pyrimidin-4-one, ainsi que ses énantiomères, diastéréoisomères et ses sels d'addition à un acide pharmaceutiquement acceptable.

10. Composés de formule (I) selon l'une quelconque des revendications 1, 3 à 6 et 8 qui sont la 3-[2-((3aα,11cα)-1,3a,4,11c-tétrahydrobenzo[5,6]chroméno[3,4-*c*]pyrrol-2(3*H*)-yl)-éthyl]-2-méthyl-4*H*-pyrido[1,2-a]pyrimidin-4-one, et la 3-[2-((3aβ,11cα)-1,3a,4,11c-tétrahydrobenzo[5,6]chroméno[3,4-*c*]pyrrol-2(3*H*)-yl)éthyl]-2-méthyl-4*H*-pyrido[1,2-*a*] pyrimidin-4-one, ainsi que leurs énantiomères et leurs sels d'addition à un acide pharmaceutiquement acceptable.

11. Procédé de préparation des composés de formule (I) selon la revendication 1, **caractérisé en ce que** l'on soumet un composé de formule (II) : 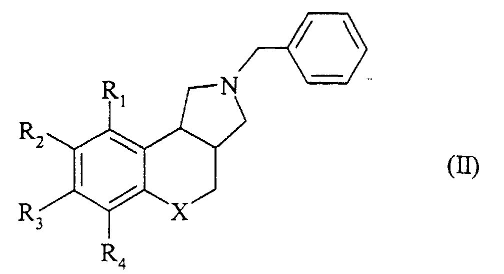 dans laquelle R₁, R₂, R₃, R₄ et X sont tels que définis dans la formule (I),
à une hydrogénation catalytique, pour conduire au composé de formule (III) : 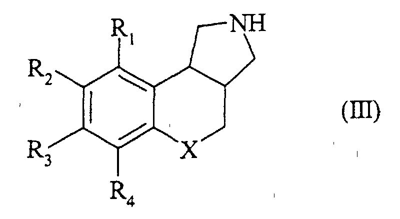 dans laquelle R₁, R₂, R₃, R₄ et X sont tels que définis précédemment,
que l'on met en réaction, soit avec un composé de formule (IV) : 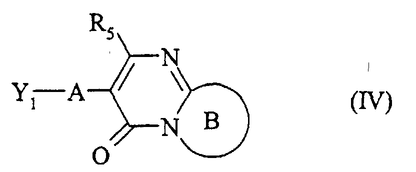 dans laquelle A, B et R₅ sont tels que définis dans la formule (I), et Y₁ représente un groupement partant tel que, par exemple, un atome d'halogène ou un groupement tosylate, triflate ou mésylate,
soit, dans des conditions d'amination réductrice, avec un composé de formule (V) : 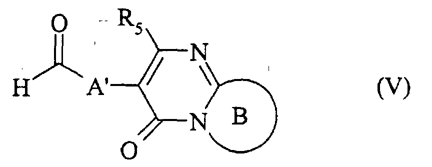 dans laquelle B et R₅ sont tels que définis dans la formule (I), et A' représente une liaison ou une chaîne alkylène (C₁-C₅) linéaire ou ramifiée,
pour conduire au composé de formule (I), que l'on purifie, le cas échéant, selon une technique classique de purification, dont on sépare, si on le souhaite, les stéréoisomères selon une technique classique de séparation, et que l'on transforme, si on le souhaite, en ses sels d'addition à un acide ou à une base pharmaceutiquement acceptable.

12. Composition pharmaceutique contenant comme principe actif un composé selon l'une quelconque des revendications 1 à 10, en combinaison avec un ou plusieurs excipients ou véhicules inertes, non toxiques, pharmaceutiquement acceptables.

13. Composition pharmaceutique selon la revendication 12 utiles en tant que médicaments antagonistes doubles α₂/5-HT_{2c}, dans le traitement de la dépression, des troubles du comportement impulsif, de l'anxiété, de la schizophrénie, de la maladie de Parkinson, des troubles cognitifs, des troubles de la libido, des dysfonctionnements sexuels, des troubles de l'appétit et des troubles du sommeil.

## Patentansprüche

1. Verbindungen der Formel (I): 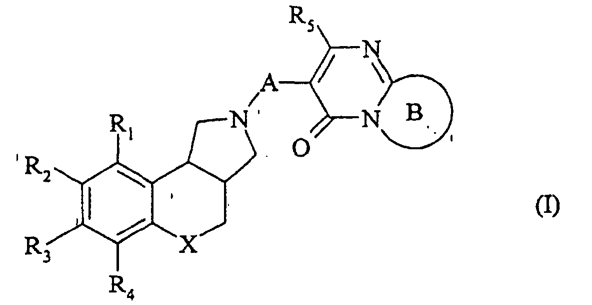 in der:
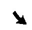 R₁, R₂, R₃ und R₃, die gleichartig oder verschieden sind, jeweils ein Wasserstoffatom, ein Halogenatom oder eine Gruppe ausgewählt aus geradkettigem oder verzweigtem (C₁-C₆)-Alkyl, geradkettigem oder verzweigtem (C₁-C₆)-Alkoxy, geradkettigem oder verzweigtem (C₁-C₆)-Polyhalogenalkyl, Hydroxy, Cyano, Nitro und Amino bedeuten.
oder R₁ mit R₂, R₂ mit R₃ oder R₃ mit R₄ gemeinsam mit den sie tragenden Kohlenstoffatomen einen gegebenenfalls substituierten Benzolring oder einen gegebenenfalls substituierten heteroaromatischen Ring bilden,
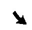 X ein Sauerstoffatom oder eine Methylengruppe bedeutet,
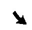 A eine geradkettige oder verzweigte (C₁-C₆)-Alkylenkette bedeutet, 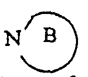
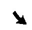 einen ungesättigten stickstoffhaltigen Heterocyclus bedeutet, der gegebenenfalls substituiert ist durch einen oder zwei gleichartige oder verschiedenartige Substituenten ausgewählt aus Halogenatomen und geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen, Cyanogruppen, Nitrogruppen, Aminogruppen, gegebenenfalls substituierten Phenylgruppen, gegebenenfalls substituierten Thienylgruppen, gegebenenfalls substituierten Furylgruppen und gegebenenfalls substituierten Pyrrolylgruppen,
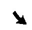 und R₅ eine geradkettige oder verzweigte (C₁-C₆)-Alkylgruppe bedeutet,
deren Enantiomere, Diastereoisomere sowie deren Additionssalze mit einer pharmazeutisch annehmbaren Säure oder Base,
wobei es sich versteht, daß:
- der Begriff (C₁-C₆)-Alkyl für eine Kohlenwasserstoffkette steht, die ein bis sechs Kohlenstoffatome enthält,
- der Begriff (C₁-C₆)-Alkoxy für eine (C₁-C₆)-Alkyloxy-gruppe steht, die ein bis sechs Kohlenstoffatome enthält,
- der Begriff (C₁-C₆)-Alkylen für eine zweiwertige Kohlenwasserstoffkette steht, die ein bis sechs Kohlenstoffatome enthält,
- der Begriff (C₁-C₆)-Polyhalogenalkyl für eine Kohlenstoff-haltige Kette steht, die ein bis sechs Kohlenstoffatome und 1 bis 9 Halogenatome enthält,
- der Begriff heteroaromatischer Ring für einen aromatischen Ring mit 5 oder 6 Kettengliedern steht, der ein, zwei oder drei Heteroatome ausgewählt aus Sauerstoff, Stickstoff und Schwefel enthält,
- der Begriff ungesättigter Stickstoff-haltiger Heterocyclus für einen ungesättigten Ring steht mit einer, zwei oder drei Unsättigungen, mit 5 bis 7 Kettengliedern und der ein, zwei oder drei Heteroatome aufweist, wobei eines dieser Heteroatome das Stickstoffatom ist, und das oder die gegebenenfalls vorhandenen zusätzlichen Heteroatome ausgewählt sind aus Sauerstoff-, Stickstoff- und Schwefelatomen,
- der Begriff gegebenenfalls substituiert, bezogen auf den Benzylring, den heteroaromatischen Ring, Phenyl, Thienyl, Furyl oder Pyrrolyl bedeutet, daß diese Gruppen gegebenenfalls substituiert sind durch einen oder zwei gleichartige oder verschiedenartige Substituenten ausgewählt aus Halogenatomen, geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen, Cyanogruppen, Nitrogruppen und Aminogruppen.

2. Verbindungen der Formel (I) nach Anspruch 1, worin R₁, R₂, R₃ und R₄, die gleichartig oder verschieden sind, jeweils ein Wasserstoffatom oder eine geradkettige oder verzweigte (C₁-C₆)-Alkylgruppe, eine geradkettige oder verzweigte (C₁-C₆)-Alkoxygruppe oder eine Hydroxygruppe bedeuten, deren Enantiomere, Diastereoisomere und deren Additionssalze mit einer pharmazeutisch annehmbaren Säure oder Base.

3. Verbindungen der Formel (I) nach Anspruch 1, worin R₁ und R₂ gemeinsam mit dem sie tragenden Kohlenstoffatom einen Benzolring bilden und R₃ und R₄ jeweils ein Wasserstoffatom darstellen, sowie deren Enantiomere, Diastereoisomere und deren Additionssalze mit einer pharmazeutisch annehmbaren Säure oder Base.

4. Verbindungen der Formel (I) nach einem der Ansprüche 1 bis 3, worin X ein Sauerstoffatom bedeutet, sowie deren Enantiomere, Diastereoisomere und deren Additionssalze mit einer pharmazeutisch annehmbaren Säure oder Base.

5. Verbindungen der Formel (I) nach einem der Ansprüche 1 bis 4, worin A eine Ethylen-, Propylen- oder Butylenkette bedeutet, sowie deren Enantiomere, Diastereoisomere und deren Additionssalze mit einer pharmazeutisch annehmbaren Säure oder Base.

6. Verbindungen der Formel (I) nach einem der Ansprüche 1 bis 5, worin 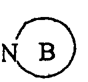 die folgende Gruppe 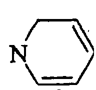 bedeutet, welche gegebenenfalls substituiert ist durch einen oder zwei gleichartige oder verschiedenartige Substituenten ausgewählt aus Halogenatomen und geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen, Cyanogruppen, Nitrogruppen, Aminogruppen und Thienylgruppen, sowie deren Enantiomere, Diastereoisomere und deren Additionssalze mit einer pharmazeutisch annehmbaren Säure oder Base.

7. Verbindungen der Formel (I) nach einem der Ansprüche 1 bis 5, worin 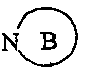 die folgende Gruppe 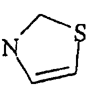 bedeutet, die gegebenenfalls substituiert ist durch ein oder zwei gleichartige oder verschiedenartige Substituenten ausgewählt aus Halogenatomen und geradkettigen oder verzweigten (C₁-C₆)-Alkylgruppen, Hydroxygruppen, geradkettigen oder verzweigten (C₁-C₆)-Alkoxygruppen, geradkettigen oder verzweigten (C₁-C₆)-Polyhalogenalkylgruppen, Cyanogruppen, Nitrogruppen und Aminogruppen, sowie deren Enantiomere, Diastereoisomere und deren Additionssalze mit einer pharmazeutisch annehmbaren Säure oder Base.

8. Verbindungen der Formel (I) nach einem der Ansprüche 1 bis 6, worin A eine Ethylenkette, X ein Sauerstoffatom, R₅ eine Methylgruppe und 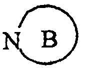 die folgende Gruppe 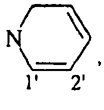 die gegebenenfalls in der 2'-Stellung durch ein Halogenatom oder eine Gruppe ausgewählt aus geradkettigem oder verzweigtem (C₁-C₆)-Alkyl, Cyano und Thienyl, bedeuten, sowie deren Enantiomere, Diastereoisomere und deren Additionssalze mit einer pharmazeutisch annehmbaren Säure oder Base.

9. Verbindung der Formel (I) nach einem der Ansprüche 1 bis 2, 4 bis 6 und 8, nämlich 3-[2-((3aα,9bα)-9-methoxy-1,3a,4,9b-tetrahydrochromeno[3,4-c]pyrrol-2(3*H*)-yl)-ethyl]-2-methyl-4*H*-pyrido[1,2-a]pyrimidin-4-on, sowie deren Enantiomere, Diastereoisomere und deren Additionssalze mit einer pharmazeutisch annehmbaren Säure oder Base.

10. Verbindungen der Formel (I) nach einem der Ansprüche 1, 3 bis 6 und 8, nämlich 3-[2-((3aα,11cα)-1,3a,4,11c-tetrahydrobenzo[5,6]chromeno[3,4-c]pyrrol-2(3*H*)-yl)-ethyl]-2-methyl-4H-pyrido[1,2-a]pyrimidin-4-on und 3-[2-((3aβ,11cα)-1,3a,4,11c-tetrahydrobenzo[5,6]chromeno[3,4-c]pyrrol-2-(3*H*)-yl)-ethyl]-2-methyl-4*H*-pyrido[1,2-*a*]pyrimidin-4-on, sowie deren Enantiomere und deren Additionssalze mit einer pharmazeutisch annehmbaren Säure oder Base.

11. Verfahren zur Herstellung der Verbindungen der Formel (I) nach Anspruch 1, **dadurch gekennzeichnet, daß** man eine Verbindung der Formel (II): 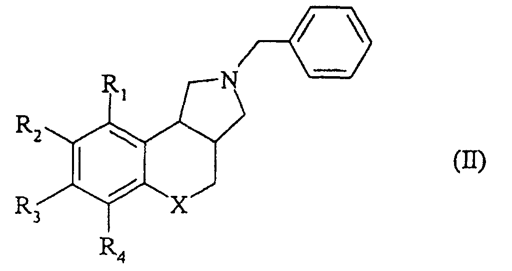 in der R₁, R₂, R₃, R₄ und X die bezüglich der Formel (I) angegebenen Bedeutungen besitzen,
einer katalytischen Hydrierung unterwirft zur Bildung der Verbindung der Formel (III): 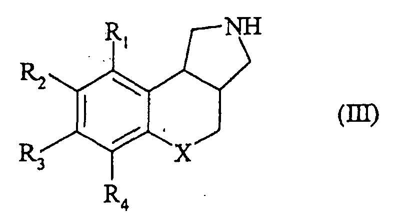 in der R₁, R₂, R₃, R₄ und X die oben angegebenen Bedeutungen besitzen, welche man entweder mit einer Verbindung der Formel (IV) umsetzt: 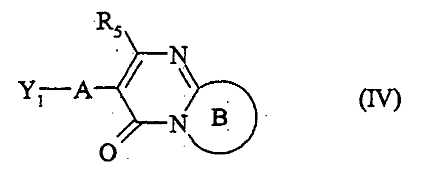 in der A, B und R₅ die bezüglich der Formel (I) angegebenen Bedeutungen besitzen und Y₁ eine austretende Gruppe bedeutet, wie beispielsweise ein Halogenatom oder eine Tosylat-, Triflat- oder Mesylatgruppe,
oder unter den Bedingungen der reduzierenden Aminierung mit einer Verbindung der Formel (V) umsetzt: 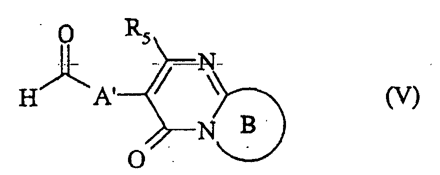 in der B und R₅ die bezüglich der Formel (I) angegebenen Bedeutungen besitzen und A' eine Bindung oder eine geradkettige oder verzweigte (C₁-C₆)-Alkylenkette bedeutet,
zur Bildung der Verbindung der Formel (I), welche man gegebenenfalls mit einer klassischen Reinigungsmethode reinigt, welche man gewünschtenfalls mit einer klassischen Trennmethode in ihre Stereoisomeren auftrennt und welche man gewünschtenfalls in ihre Additionssalze mit einer pharmazeutisch annehmbaren Säure oder Base überführt.

12. Pharmazeutische Zubereitung enthaltend als Wirkstoff eine Verbindung nach einem der Ansprüche 1 bis 10 in Kombination mit einem oder mehreren inerten, nichttoxischen, pharmazeutisch annehmbaren Trägermaterialien oder Hilfsstoffen.

13. Pharmazeutische Zubereitung nach Anspruch 12, die nützlich ist als doppelt antagonistisches α₂/5-HT_{2c}-Arzneimittel bei der Behandlung von Depressionen, Störungen des impulsiven Verhaltens, der Angst, der Schizophrenie, der Parkinsonschen Krankheit, von Erkenntnisstörungen, von Libidostörungen, von sexuellen Dysfunktionen, Appetitstörungen und Schlafstörungen.

## Claims

1. Compounds of formula (I) : 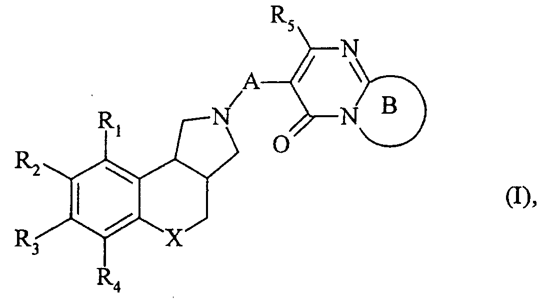 wherein :
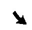 R₁, R₂, R₃ and R₄, which may be the same or different, each represent a hydrogen atom, a halogen atom or a group selected from linear or branched (C₁-C₆)alkyl, linear or branched (C₁-C₆)alkoxy, linear or branched (C₁-C₆)polyhaloalkyl, hydroxy, cyano, nitro and amino,
or R₁ with R₂, R₂ with R₃, or R₃ with R₄, together with the carbon atoms carrying them, form an optionally substituted benzene ring or an optionally substituted heteroaromatic ring,
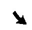 X represents an oxygen atom or a methylene group,
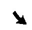 A represents a linear or branched (C₁-C₆)alkylene chain, 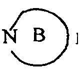 represents an unsaturated nitrogen-containing heterocycle optionally substituted by one or two identical or different substituents selected from halogen atoms and linear or branched (C₁-C₆)alkyl, hydroxy, linear or branched (C₁-C₆)alkoxy, linear or branched (C₁-C₆)polyhaloalkyl, cyano, nitro, amino, optionally substituted phenyl, optionally substituted thienyl, optionally substituted furyl and optionally substituted pyrrolyl groups,
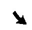 and R₅ represents a linear or branched (C₁-C₆)alkyl group,
their enantiomers and diastereoisomers, and addition salts thereof with a pharmaceutically acceptable acid or base,
it being understood that :
- the term "(C₁-C₆)alkyl" denotes a hydrocarbon chain containing from one to six carbon atoms,
- the term "(C₁-C₆)alkoxy" denotes a (C₁-C₆)alkyl-oxy group containing from one to six carbon atoms,
- the term "(C₁-C₆)alkylene" denotes a bivalent hydrocarbon chain containing from one to six carbon atoms,
- the term (C₁-C₆)polyhaloalkyl denotes a carbon chain containing from one to six carbon atoms and from 1 to 9 halogen atoms,
- the term "heteroaromatic ring" denotes a 5- or 6-membered aromatic ring containing one, two or three hetero atoms selected from oxygen, nitrogen and sulphur,
- the term "unsaturated nitrogen-containing heterocycle" denotes a 5- to 7-membered unsaturated ring having one, two or three unsaturated bonds and containing one, two or three hetero atoms, one of those hetero atoms being the nitrogen atom and the additional hetero atom(s) optionally present being selected from oxygen, nitrogen and sulphur atoms,
- the term "optionally substituted" applied to the expressions "benzene ring", "heteroaromatic ring", "phenyl", "thienyl", "furyl" or pyrrolyl" indicates that those groups are optionally substituted by one or two identical or different substituents selected from halogen atoms and linear or branched (C₁-C₆)alkyl, hydroxy, linear or branched (C₁-C₆)alkoxy, linear or branched (C₁-C₆)polyhaloalkyl, cyano, nitro and amino groups.

2. Compounds of formula (I) according to claim 1, wherein R₁, R₂, R₃ and R₄, which may be the same or different, each represent a hydrogen atom or a linear or branched (C₁-C₆)alkyl, linear or branched (C₁-C₆)alkoxy or hydroxy group, their enantiomers and diastereoisomers, and addition salts thereof with a pharmaceutically acceptable acid or base.

3. Compounds of formula (I) according to claim 1, wherein R₁ and R₂, together with the carbon atoms carrying them, form a benzene ring and R₃ and R₄ each represent a hydrogen atom, their enantiomers and diastereoisomers, and addition salts thereof with a pharmaceutically acceptable acid or base.

4. Compounds of formula (I) according to any one of claims 1 to 3, wherein X represents an oxygen atom, their enantiomers and diastereoisomers, and addition salts thereof with a pharmaceutically acceptable acid or base.

5. Compounds of formula (I) according to any one of claims 1 to 4, wherein A represents an ethylene, propylene or butylene chain, their enantiomers and diastereoisomers, and addition salts thereof with a pharmaceutically acceptable acid or base.

6. Compounds of formula (I) according to any one of claims 1 to 5, wherein 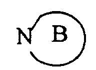 represents the following group 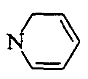 optionally substituted by one or two identical or different substituents selected from halogen atoms and linear or branched (C₁-C₆)alkyl, hydroxy, linear or branched (C₁-C₆)alkoxy, linear or branched (C₁-C₆)polyhaloalkyl, cyano, nitro, amino and thienyl groups, their enantiomers and diastereoisomers, and addition salts thereof with a pharmaceutically acceptable acid or base.

7. Compounds of formula (I) according to any one of claims 1 to 5, wherein 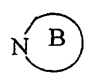 represents the following group 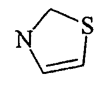 optionally substituted by one or two identical or different substituents selected from halogen atoms and linear or branched (C₁-C₆)alkyl, hydroxy, linear or branched (C₁-C₆)alkoxy, linear or branched (C₁-C₆)polyhaloalkyl, cyano, nitro and amino groups, their enantiomers and diastereoisomers, and addition salts thereof with a pharmaceutically acceptable acid or base.

8. Compounds of formula (I) according to any one of claims 1 to 6, wherein A represents an ethylene chain, X represents an oxygen atom, R₅ represents a methyl group and 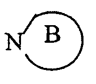 represents the following group 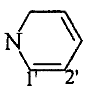 optionally substituted in the 2'-position by a halogen atom or a group selected from linear or branched (C₁-C₆)alkyl, cyano and thienyl, their enantiomers and diastereoisomers, and addition salts thereof with a pharmaceutically acceptable acid or base.

9. Compound of formula (I) according to any one of claims 1 to 2, 4 to 6 and 8, which is 3-[2-((3aα,9bα)-9-methoxy-1,3a,4,9b-tetrahydrochromeno[3,4-*c*]pyrrol-2(3*H*-yl)-ethyl]-2-methyl-4*H*-pyrido[1,2-*a*]pyrimidin-4-one, its enantiomers and diastereoisomers, and addition salts thereof with a pharmaceutically acceptable acid.

10. Compounds of formula (I) according to any one of claims 1, 3 to 6 and 8, which are 3-[2-((3aα,11cα)-1,3a,4,11c-tetrahydrobenzo[5,6]chromeno[3,4-*c*]pyrrol-2(3*H*)-yl)-ethyl]-2-methyl-4*H*-pyrido[1,2-a]pyrimidin-4-one and 3-[2-((3aβ,11cα)-1,3a,4,11c-tetrahydrobenzo[5,6]chromeno[3,4-*c*]pyrrol-2(3*H*)-yl)ethyl]-2-methyl-4*H*-pyrido[1,2-*a*]pyrimidin-4-one, their enantiomers, and addition salts thereof with a pharmaceutically acceptable acid.

11. Process for the preparation of compounds of formula (I) according to claim 1,
**characterised in that** a compound of formula (II) : 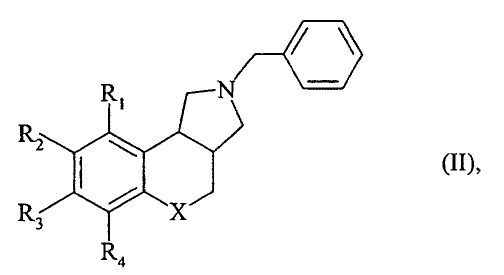 wherein R₁, R₂, R₃, R₄ and X are as defined for formula (I),
is subjected to catalytic hydrogenation to yield the compound of formula (III) : 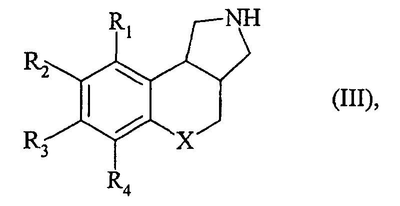 wherein R₁, R₂, R₃, R₄ and X are as defined hereinbefore,
which is reacted either with a compound of formula (IV) : 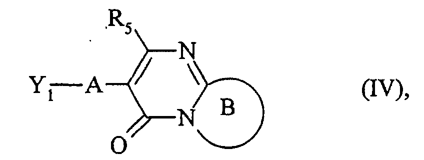 wherein A, B and R₅ are as defined for formula (I) and Y₁ represents a leaving group such as, for example, a halogen atom or a tosylate, triflate or mesylate group,
or, under conditions of reductive amination, with a compound of formula (V) : 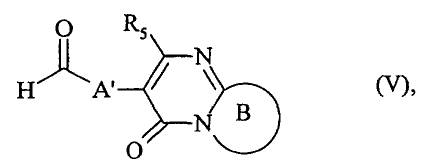 wherein B and R₅ are as defined for formula (I) and A' represents a bond or a linear or branched (C₁-C₅)alkylene chain,
to yield the compound of formula (I), which is purified, if necessary, according to a conventional purification technique, which is separated, if desired, into its stereoisomers according to a conventional separation technique, and which is converted, if desired, into addition salts thereof with a pharmaceutically acceptable acid or base.

12. Pharmaceutical composition comprising as active ingredient a compound according to any one of claims 1 to 10, in combination with one or more inert, non-toxic, pharmaceutically acceptable excipients or carriers.

13. Pharmaceutical composition according to claim 12 for use as a double α₂/5-HT_{2c} antagonist medicament in the treatment of depression, impulsive behaviour disorders, anxiety, schizophrenia, Parkinson's disease, cognitive disorders, disturbances of libido, sexual dysfunctions, appetite disorders and sleep disorders.
